# EUROPEAN PATENT APPLICATION

(11) **EP 1 459 754 A1**
(43) Date of publication of application: **22.09.2004**
(21) Application number: 02793414.0
(22) Date of filing: 26.12.2002
(51) Int. Cl.: A61K 38/00, A61K 31/7088, A61K 45/00, A61K 48/00, A61P 35/00

(54) **PREVENTIVES/REMEDIES FOR CANCER**

(30) Priority: 27.12.2001 JP 2001398220
(71) Applicant: Takeda Chemical Industries, Ltd., Osaka-shi, Osaka 541-0045 (JP)
(72) Inventor: HIKICHI, Yuichi, Shareru Tsukuba Matsushiro 1-504, Tsukuba-shi, Ibaraki 305-0035 (JP); KATSUYAMA, Ryosuke, Takeda Matsushiro Residence, Tsukuba-shi, Ibaraki 305-0035 (JP); KAKOI, Yuichi, Tsukuba-shi, Ibaraki 300-2635 (JP)
(74) Representative: Rickard, Timothy Mark Adrian
(86) International application number: PCT/JP2002/013640
(87) International publication number: WO 2003/055506

(57) **Abstract**

A compound or its salt that regulates the activities of a protein having the same or substantially the same amino acid sequence as the amino acid sequence represented by SEQ ID NO: 1, an antisense nucleotide containing a base sequence or a part thereof which is complementary or substantially complementary to the base sequence of a DNA encoding a protein or its partial peptide having the same or substantially the same amino acid sequence as the amino acid sequence represented by SEQ ID NO: 1, etc. can be used as preventive/therapeutic agents for cancer, etc.

## Description

### FIELD OF THE INVENTION

The present invention relates to preventives/remedies and diagnostics for cancer, etc.

### BACKGROUND ART

In cancer chemotherapy, the development of new anticancer drugs results in improved life-extending effects, which increases cases moving toward cure. However, almost all anticancer drugs currently used cause damages on DNA and exert potent cytotoxicity to arrest cell division. For these reasons, anticancer drugs considerably injure normal cells and strong side-effects often appear especially on the bone marrow with vigorous cell division.

For exhaustive analysis of gene expression, a microarray analysis using immobilized cDNA or oligonucleotide was developed so that techniques of detecting changes in disease-specific gene expression became popular and its benefits have been confirmed. For example, the GeneChip system of Affymetrix Inc. is in heavy use for diagnosis of diseases such as cancer and finding of drug development target genes.

Antisense oligonucleotides, when transfected to cells, hybridize to RNA having complementary sequence and induce degradation of RNA by RNase H, inhibiting protein translation or causing inhibition of direct protein synthesis by hybridization. Since it is possible to specifically prevent functions of the objective gene, antisense oligonucleotides are frequently used as a means for analyzing gene functions and in some of them, development is advancing on clinical applications.

In recent years it gradually became clear that chromatin structure is deeply involved in regulating the division and growth of cells or the transcription of genes. In histones which constitute chromatin, it is known that especially the domains called histone tails undergo modifications such as acetylation, phosphorylation or methylation to contribute to change in the structure of chromatin (JIKKEN IGAKU, edited by Nakatani et al., October 2001).

Suppressor of variegation 3-9 homolog 1 (hereinafter sometimes referred to as SUV39H1) is a gene isolated as a human homolog of position effect variegation (PEV) suppressor gene Su(var)3-9 of Drosophila (EMBO.J., 18, 1923-1938, 1999). SUV39H1 is a histone methyltransferase that provides methyl group specifically for lysine-9 of the N terminus of histone H3 protein, and takes part in forming the chromatin structure. Reportedly, lysine that undergoes methyl transfer by SUV39H1 is further modified by acetylation, in addition to methylation, and by the action of each transferase (histone methyltransferase and histone acetyltransferase) or a detransferase, these two competitively bind to the lysine residue (Nature, 406, 593-599, 2000).

On the other hand, there are reports on a transgenic mice (Mech. Dev. 107 (1-2), 141-153, 2001) and knockout mice (Cell 107, 323-337, 2001) but any significant change in phenotype is not reported, except that some growth retardation and erythroid differentiation were noted. In order to study the functions of SUV39H1, Nielsen et al. investigated effects of SUV39H1 on cyclin E promoter activities, using a vector inserted with the cyclin E promoter upstream the reporter gene. As a result, they reported that SUV39H1 co-expressed with Rb1 to repress the cyclin E promoter activities (Nature 412, 561-565, 2001). Based on these results, the role of SUV39H1 was recognized rather as a tumor suppressor gene until recently (Nature Reviews Cancer 2, 469-476, 2002) but it was not considered that SUV39H1 would positively be involved in malignant transformation. In recent years, histone deacetyltransferase was targeted and development of anticancer drugs to inhibit its functions has been advanced, but those targeting histone methyltransferase have not been reported so far.

### DISCLOSURE OF THE INVENTION

It has been desired earnestly to develop drugs targeting molecules that specifically express on cancer cells to inhibit growth of cancer cells or induce apoptosis.

In order to solve the foregoing problems, the present inventors have made extensive studies and as a result, found a gene, which expression markedly increases on cancerous tissues. Based on this finding, further studies have been made and the present invention has come to be accomplished.

That is, the present invention provides the following features.
(1) A preventive/therapeutic agent for cancer, comprising a compound or its salt that inhibits the activities of a protein containing the same or substantially the same amino acid sequence as the amino acid sequence represented by SEQ ID NO: 1, its partial peptide or a salt thereof;
(2) A preventive/therapeutic agent for cancer, comprising a compound or its salt that inhibits the expression of a gene for a protein containing the same or substantially the same amino acid sequence as the amino acid sequence represented by SEQ ID NO: 1, its partial peptide or a salt thereof;
(3) An antisense nucleotide containing a base sequence complementary or substantially complementary to the base sequence of a DNA encoding a protein having the same or substantially the same amino acid sequence as the amino acid sequence represented by SEQ ID NO: 1, its partial peptide or a salt thereof, or a part of the base sequence;
(4) A preventive/therapeutic agent for cancer, comprising the antisense nucleotide according to (3);
(5) A preventive/therapeutic agent for cancer, comprising an antibody to a protein containing the same or substantially the same amino acid sequence as the amino acid sequence represented by SEQ ID NO: 1, its partial peptide or a salt thereof;
(6) The preventive/therapeutic agent for cancer according to (1) through (5), wherein the cancer is colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor;
(7) A diagnostic product comprising an antibody to a protein containing the same or substantially the same amino acid sequence as the amino acid sequence represented by SEQ ID NO: 1, its partial peptide or a salt thereof;
(8) A diagnostic product comprising a DNA encoding a protein containing the same or substantially the same amino acid sequence as the amino acid sequence represented by SEQ ID NO: 1, its partial peptide or a salt thereof;
(9) The diagnostic product according to (7) or (8), wherein the cancer is colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor;
(10) A preventive/therapeutic agent for cancer, comprising a compound having a regulatory action on the histone methyltransferase activity, or a salt thereof;
(11) A preventive/therapeutic agent for cancer, comprising a compound having a histone methyltransferase expression regulatory action, or a salt thereof;
(12) A method of screening a preventive/therapeutic agent for cancer, which comprises using a protein containing the same or substantially the same amino acid sequence as the amino acid sequence represented by SEQ ID NO: 1, its partial peptide or a salt thereof;
(13) A kit for screening a preventive/therapeutic agent for cancer, comprising a protein containing the same or substantially the same amino acid sequence as the amino acid sequence represented by SEQ ID NO: 1, its partial peptide or a salt thereof;
(14) A method of screening a preventive/therapeutic agent for cancer, which comprises using a DNA encoding a protein containing the same or substantially the same amino acid sequence as the amino acid sequence represented by SEQ ID NO: I, its partial peptide or a salt thereof;
(15) A kit for screening a preventive/therapeutic agent for cancer, comprising a DNA encoding a protein containing the same or substantially the same amino acid sequence as the amino acid sequence represented by SEQ ID NO: 1, its partial peptide or a salt thereof;
(16) A preventive/therapeutic agent for cancer, which is obtainable by using the screening method according to (12) or (14) or the screening kit according to (13) or (15);
(17) An apoptosis inducer comprising a compound or its salt that inhibits the activities of a protein containing the same or substantially the same amino acid sequence as the amino acid sequence represented by SEQ ID NO: 1, its partial peptide or a salt thereof;
(18) An apoptosis inducer comprising a compound or its salt that inhibits the expression of a gene for a protein containing the same or substantially the same amino acid sequence as the amino acid sequence represented by SEQ ID NO: 1, its partial peptide or a salt thereof;
(19) A method of screening an apoptosis inducer, which comprises using a protein containing the same or substantially the same amino acid sequence as the amino acid sequence represented by SEQ ID NO: 1, its partial peptide or a salt thereof;
(20) A method of screening an apoptosis inducer, which comprises using a DNA encoding a protein containing the same or substantially the same amino acid sequence as the amino acid sequence represented by SEQ ID NO: 1, its partial peptide or a salt thereof;
(21) A method of preventing/treating cancer, which comprises administering to a mammal an effective amount of a compound or its salt that inhibits the activities of a protein containing the same or substantially the same amino acid sequence as the amino acid sequence represented by SEQ ID NO: 1, its partial peptide or a salt thereof, or a compound or its salt that inhibits the expression of a gene for the protein, its partial peptide or a salt thereof;
(22) Use of a compound or its salt that inhibits the activities of a protein containing the same or substantially the same amino acid sequence as the amino acid sequence represented by SEQ ID NO: 1, its partial peptide or a salt thereof, or a compound or its salt that inhibits the expression of a gene for the protein, its partial peptide or a salt thereof; and the like.

### PREFERRED EMBODIMENT OF THE INVENTION

The protein containing the same or substantially the same amino acid sequence as the amino acid sequence represented by SEQ ID NO: 1, which is used in the present invention (hereinafter sometimes referred to as the protein of the present invention or the protein used in the present invention) may be any protein derived from any cells of human and warm-blooded animals (e.g., guinea pig, rat, mouse, fowl, rabbit, swine, sheep, bovine, monkey, etc.) such as hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophage, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.), or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and. small intestine), blood vessel, heart, thymus, spleen, submandibular gland, peripheral blood, prostate, testis, ovary, placenta, uterus, bone, joint, skeletal muscle, etc.; the protein may also be a synthetic protein.

The amino acid sequence which is substantially the same as the amino acid sequence represented by SEQ ID NO: 1 includes amino acid sequences having at least about 50% homology, preferably at least about 60% homology, more preferably at least about 70% homology, further more preferably about 80% homology, much more preferably about 90% homology and most preferably at least about 95% homology, to the amino acid sequence represented by SEQ ID NO: 1; etc.

Preferred examples of the protein containing substantially the same amino acid sequence as the amino acid sequence represented by SEQ ID NO: 1 include proteins having substantially the same amino acid sequence as the amino acid sequence represented by SEQ ID NO: 1 and having activities substantially equivalent to those of the protein containing the amino acid sequence represented by SEQ ID NO: 1, etc.

The substantially equivalent activities include, for example, a histone methyltransferase activity and the like. The substantially equivalent is used to mean that the nature of these properties is equivalent in terms of quality (e.g., physiologically or pharmacologically). Thus, the histone methyltransferase activity is preferably equivalent (e.g., about 0.01 to 100 times, preferably about 0.1 to 10 times, more preferably 0.5 to 2 times), but differences in degree such as a level of these activities, quantitative factors such as a molecular weight of the protein may be present and allowable.

The histone methyltransferase activity can be assayed by methods per se publicly known, for example, the method described in Nature, 406, 593-599, 2000, or its modifications. More specifically, the protein of the present invention is reacted with S-adenosyl-L-methionine wherein the methyl group is radio-labeled and a histone protein or a polypeptide having the N-terminal sequence of histone H3, and the radioactivity of histone H3 or the polypeptide by transfer of the methyl group is assayed. The reaction is carried out in an appropriate buffer. After the enzymatic reaction, the reaction product is separated, e.g., by SDS-PAGE, etc. and compared with the mobility of histone H3 or the like as a standard control to perform identification. The radioactivity for quantification is assayed in accordance with publicly known methods using a scintillation counter, fluorography, etc.

Examples of the protein used in the present invention include so-called muteins such as proteins containing (1) the amino acid sequence represented by SEQ ID NO: 1, of which at least 1 or 2 (e.g., about 1 to about 100, preferably about 1 to about 30, preferably about 1 to about 10 and more preferably several (1 to 5)) amino acids are deleted; (2) the amino acid sequence represented by SEQ ID NO: 1, to which at least 1 or 2 (e.g., about 1 to about 100, preferably about 1 to about 30, preferably about 1 to about 10 and more preferably several (1 to 5)) amino acids are added; (3) the amino acid sequence represented by SEQ ID NO: 1, in which at least 1 or 2 (e.g., about 1 to about 100, preferably about 1 to about 30, preferably about 1 to about 10 and more preferably several (1 to 5)) amino acids are inserted; (4) the amino acid sequence represented by SEQ ID NO: 1, in which at least 1 or 2 (e.g., about 1 to about 100, preferably about 1 to about 30, preferably about 1 to about 10 and more preferably several (1 to 5)) amino acids are substituted by other amino acids; or (5) a combination of these amino acid sequences; etc.

When the amino acid sequence is inserted, deleted or substituted as described above, positions of the insertion, deletion or substitution are not particularly limited.

Throughout the specification, the proteins are represented in accordance with the conventional way of describing proteins, that is, the N-terminus (amino terminus) at the left hand and the C-terminus (carboxyl terminus) at the right hand. In the protein used in the present invention including the protein containing the amino acid sequence shown by SEQ ID NO: 1, the C-terminus may be in any form of a carboxyl group (-COOH), a carboxylate (-COO-), an amide (-CONH₂) or an ester (-COOR).

Herein, examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, alpha-naphthyl, etc.; a C₇₋₁₄ aralkyl such as a phenyl-C₁₋₂ alkyl group, e.g., benzyl, phenethyl, etc.; an alpha-naphthyl-C₁₋₂ alkyl group such as alpha-naphthylmethyl, etc.; pivaloyloxymethyl and the like.

Where the protein used in the present invention contains a carboxyl group (or a carboxylate) at a position other than the C-terminus, the carboxyl group may be amidated or esterified and such an amide or ester is also included within the protein used in the present invention. Examples of the ester group in this case may be the C-terminal esters described above, etc.

Furthermore, examples of the protein used in the present invention include variants wherein the amino group at the N-terminal amino acid residues (e.g., methionine residue) is protected with a protecting group (e.g., a C₁₋₆ acyl group such as a C₁₋₆ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₁₋₆ alkanoyl group, e.g., formyl group, acetyl group, etc.), or conjugated proteins such as glycoproteins having sugar chains; etc.

Specific examples of the protein used in the present invention are a protein (SUV39H1) containing the amino acid sequence represented by SEQ ID NO: 1, and the like.

The partial peptide of the protein used in the present invention may be any peptide as long as it is a partial peptide of the protein used in the present invention described above and preferably has the property equivalent to that of the protein used in the present invention described above.

Specifically, for the purpose of preparing the antibody of the present invention later described, a peptide having the sequence of 42nd to 366th amino acid residues in the amino acid sequence represented by SEQ ID NO: 1, and the like, are used. For example, there are employed peptides containing the sequence of, e.g., at least 20, preferably at least 50, more preferably at least 70, much more preferably at least 100, and most preferably at least 200, amino acids in the constituent amino acid sequence of the protein used in the present invention, and the like.

The partial peptide used in the present invention may be peptides containing the amino acid sequence, of which at least 1 or 2 (preferably about 1 to about 20, more preferably about 1 to about 10 and most preferably several (1 to 5)) amino acids may be deleted; or peptides, to which at least 1 or 2 (preferably about 1 to about 20, more preferably about 1 to about 10 and most preferably several (1 to 5)) amino acids may be added; or peptides, in which at least 1 or 2 (preferably about 1 to about 20, more preferably about 1 to about 10 and most preferably several (1 to 5)) amino acids may be inserted; or peptides, in which at least 1 or 2 (preferably about 1 to about 20, more preferably about 1 to about 10 and most preferably several (1 to 5)) amino acids may be substituted by other amino acids.

In the partial peptide used in the present invention, the C-terminus may be in any form of a carboxyl group (-COOH), a carboxylate (-COO-), an amide (-CONH₂) or an ester (-COOR).

Furthermore, the partial peptide used in the present invention includes variants having a carboxyl group (or a carboxylate) at a position other than the C-terminus, those wherein the amino group at the N-terminal amino acid residues (e.g., methionine residue) is protected with a protecting group; those wherein the N-terminal region is cleaved in vivo and the glutamyl group thus formed is pyroglutaminated; those wherein a substituent on the side chain of an amino acid in the molecule is protected with a suitable protecting group, or conjugated proteins such as so-called glycoproteins having sugar chains; etc., as in the proteins used in the present invention described above.

The partial peptide used in the present invention may also be used as an antigen for producing antibodies.

As salts of the protein or partial peptide used in the present invention, salts with physiologically acceptable acids (e.g., inorganic acids or organic acids) or bases (e.g., alkali metal salts) may be employed, preferably in the form of physiologically acceptable acid addition salts. Examples of such salts include salts with inorganic acids (e.g., hydrochloric acid, phosphoric acid, hydrobromic acid, and sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein or partial peptide used in the present invention or salts thereof may be manufactured by publicly known methods used to purify a protein from human or warm-blooded animal cells or tissues described above. Alternatively, they may also be manufactured by culturing transformants containing DNAs encoding these proteins. Furthermore, they may also be manufactured by a modification of the methods for peptide synthesis, which will be described later.

Where these proteins are manufactured from human or mammalian tissues or cells, human or mammalian tissues or cells are homogenized, extracted with an acid or the like, and the extract can be isolated and purified by a combination of chromatography techniques such as reverse phase chromatography, ion exchange chromatography, and the like.

To synthesize the protein or partial peptide used in the present invention or its salts, or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine, resin, PAM, resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids, in which alpha-amino groups and functional groups on the side chains are appropriately protected, are condensed on the resin in the order of the sequence of the objective protein according to various condensation methods publicly known in the art. At the end of the reaction, the protein or partial peptide is excised from the resin and at the same time, the protecting groups are removed. Then, intramolecular disulfide bond-forming reaction is performed in a highly diluted solution to obtain the objective protein or partial peptide, or amides thereof.

For condensation of the protected amino acids described above, a variety of activation reagents for protein synthesis may be used, and carbodiimides are particularly employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20 °C to 50 °C . The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g:, alkyl esterification (linear, branched or cyclic alkyl esterification of, e.g., methyl, ethyl, propyl, butyl, t-butyl, cyclopentyl, cyclohexyl, cycloheptyl, cyclooctyl, 2-adamantyl, etc.), aralkyl esterification (e.g., benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower (C₁₋₆) alkanoyl group, such as acetyl group; an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, or a mixture solution of these acids; a treatment with a base such as diisopropylethylamine, triethylamine, piperidine or piperazine; reduction with sodium in liquid ammonia, etc. The elimination of the protecting group by the acid treatment described above is carried out generally at a temperature of approximately -20 °C to 40 °C . In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the desired protein or partial peptide, for example, the alpha-carboxyl group of the carboxy terminal amino acid is first protected by amidation; the peptide (protein) chain is then extended from the amino group side to a desired length. Thereafter, a protein or partial peptide, in which only the protecting group of the N-terminal alpha-amino group of the peptide chain has been eliminated, and a protein or partial peptide, in which only the protecting group of the C-terminal carboxyl group has been eliminated are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the alpha-carboxyl group of the carboxy terminal amino acid is condensed with a desired alcohol to prepare the amino acid ester, which is followed: by procedures similar to the preparation of the amidated protein or peptide above to give the desired esterified protein or peptide.

The partial peptide used in the present invention or salts thereof can be manufactured by publicly known methods for peptide synthesis, or by cleaving the protein used in the present invention with an appropriate peptidase. For the methods for peptide synthesis, for example, either solid phase synthesis or liquid phase synthesis may be used. That is, the partial peptide or amino acids that can construct the partial peptide used in the present invention are condensed with the remaining part. Where the product contains protecting groups, these protecting groups are removed to give the desired peptide. Publicly known methods for condensation and elimination of the protecting groups are described in (1) to (5) below.
(1) M. Bodanszky & M.A. Ondetti: Peptide Synthesis, Interscience Publishers, New York (1966)
(2) Schroeder & Luebke: The Peptide, Academic Press, New York (1965)
(3) Nobuo Izumiya, et al.: Peptide Gosei-no-Kiso to Jikken (Basics and experiments of peptide synthesis), published by Maruzen Co. (1975)
(4) Haruaki Yajima & Shunpei Sakakibara:: Seikagaku Jikken Koza (Biochemical Experiment) 1, Tanpakushitsu no Kagaku (Chemistry of Proteins) IV, 205 (1977)
(5) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide used in the present invention. When the partial peptide obtained by the above methods is in a free form, the partial peptide can be converted into an appropriate salt by a publicly known method; when the partial peptide is obtained in a salt form, it can be converted into a free form or other different salt form by a publicly known method.

The polynucleotide encoding the protein used in the present invention may be any polynucleotide so long as it contains the base sequence encoding the protein used in the present invention described above. Preferably, the polynucleotide is a DNA. The DNA may be any of genomic DNA, genomic DNA library, cDNA derived from the cells or tissues described above, cDNA library derived from the cells or tissues described above and synthetic DNA.

The vector used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

The DNA encoding the protein used in the present invention may be any DNA containing the base sequence represented by, for example, SEQ ID NO: 2, or any DNA containing a base sequence hybridizable to a DNA having the base sequence represented by SEQ ID NO: 2 under high stringent conditions and encoding a protein which has the properties substantially equivalent to those of the protein containing the amino acid sequence represented by SEQ ID NO: 1.

As the DNA that is hybridizable to the base sequence represented by SEQ ID NO: 2 under high stringent conditions, there are employed, for example, DNAs having at least about 50% homology, preferably at least about 60% homology, more preferably at least about 70% homology, more preferably at least about 80% homology, particularly preferably at least about 90% homology and most preferably at least about 95% homology, to the base sequence represented by SEQ ID NO: 2.

The hybridization can be carried out by per se publicly known methods or by modifications thereof, for example, according to the method described in Molecular Cloning, 2nd. (J. Sambrook et al., Cold Spring Harbor Lab. Press, 1989), etc. A commercially available library can also be used according to the instructions of the attached manufacturer's protocol. More preferably, the hybridization can be carried out under high stringent conditions.

The high stringent conditions are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.

More specifically, as the DNA encoding the protein containing the amino acid sequence represented by SEQ ID NO: 1, there may be employed a DNA containing the base sequence represented by SEQ ID NO: 2, etc.

The DNA encoding the partial peptide used in the present invention may be any DNA so long as it contains the base sequence encoding the partial peptide used in the present invention described above. The DNA may also be any of genomic DNA, genomic DNA library, cDNA derived from the cells and tissues described above, cDNA library derived from the cells and tissues described above and synthetic DNA.

As the DNA encoding the partial peptide used in the present invention, there are employed, for example, a DNA having a part of DNA containing the base sequence represented by SEQ ID NO: 2, or a DNA containing a base sequence hybridizable to the base sequence represented by SEQ ID NO: 2 under high stringent conditions and containing a part of DNA encoding a protein having the activities substantially equivalent to those of the protein of the present invention, etc.

The DNA hybridizable to the base sequence represented by SEQ ID NO: 2 has the same significance as described above.

Methods for the hybridization and the high stringent conditions that can be used are the same as those described above.

For cloning of DNAs that completely encode the protein or partial peptide used in the present invention (hereinafter sometimes merely referred to as the protein of the present invention in describing the cloning of DNAs encoding the protein and partial peptide and their expression), the DNA can be either amplified by PCR using synthetic DNA primers containing a part of the base sequence of the protein of the present invention, or the DNA inserted into an appropriate vector can be screened by hybridization with a labeled DNA fragment or synthetic DNA that encodes a part or entire region of the protein of the present invention. The hybridization can be carried out, for example, according to the method described in Molecular Cloning, 2nd (J. Sambrook et al., Cold Spring Harbor Lab. Press, 1989). Where the hybridization is carried out using commercially available library, the procedures may be conducted in accordance with the protocol described in the attached instructions.

Conversion of the base sequence of DNA can be effected by publicly known methods such as the ODA-LA PCR method, the Gapped duplex method, the Kunkel method, etc., or its modifications, using a publicly known kit available as Mutan™-super Express Km (manufactured by Takara Shuzo Co.; Ltd.) or Mutan™-K (manufactured by Takara Shuzo Co., Ltd.), etc.

The cloned DNA encoding the protein can be used as it is, depending upon purpose or after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the protein of the present invention can be manufactured, for example, by (a) excising the desired DNA fragment from the DNA encoding the protein of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNA I/Neo, etc.

The promoter used in the present invention may be any promoter if it matches well with a host to be used for gene expression. In the case of using animal cells as the host, examples of the promoter include SR α promoter, SV40 promoter, LTR promoter, CMV promoter, HSV-TK promoter, etc.

Among them, it is preferred to use CMV (cytomegalovirus) promoter, SR α promoter, etc. Where the host is bacteria of the genus Escherichia, preferred examples of the promoter include trp promoter, lac promoter, recA promoter, λP_{L} promoter, lpp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker using dhfr gene-deficient Chinese hamster cells, the objective gene can also be selected on a thymidine free medium.

If necessary, a signal sequence that matches with a host is added to the N-terminus of the protein of the present invention. Examples of the signal sequence that can be used are PhoA signal sequence, OmpA signal sequence, etc. when bacteria of the genus Escherichia is used as the host; α-amylase signal sequence, subtilisin signal sequence, etc. when bacteria of the genus Bacillus is used as the host; MFα signal sequence; SUC2 signal sequence, etc. when yeast is used as the host; and insulin signal sequence, alpha-interferon signal sequence, antibody molecule signal sequence, etc. when animal cells are used as the host, respectively.

Using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977), etc.

As the insect, for example, a larva of Bombyx mori can be used [Maeda et al., Nature, 315, 592 (1985)].

Examples of animal cells include monkey cell COS-7, Vero, Chinese hamster cell CHO (hereinafter referred to as CHO cell), dhfr gene-deficient Chinese hamster cell CHO (hereinafter simply referred to as CHO (dhfr⁻) cell), mouse L cell, mouse AtT-20, mouse myeloma cell, rat GH 3, human FL cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics; 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformants transformed with the expression vectors containing the DNAs encoding the protein of the present invention can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultured generally at about 30 to 40°C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to 8. In general, the transformant is cultivated at about 20 to 35°C for about 24 to 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added: Preferably, pH of the medium is adjusted to about 6.2 to about 6.4. Normally, the transformant is cultivated at about 27°C for about 3 days to about 5 days and, if necessary; the culture can be aerated or agitated.

Where animal cells are employed as the host, the transformant is cultured in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 to 60 hours and, if necessary, the culture can be aerated or agitated.

As described above, the protein of the present invention can be produced in the transformant, in the cell membrane of the transformant, or outside of the transformant.

The protein of the present invention can be separated and purified from the culture described above by the following procedures.

When the protein of the present invention is extracted from the bacteria or cells, the bacteria or cell is collected after culturing by a publicly known method and suspended in an appropriate buffer. The bacteria or cell is then disrupted by publicly known methods such as ultrasonication, a treatment with lysozyme and/or freeze-thaw cycling, followed by centrifugation, filtration, etc. Thus, the crude extract of the protein can be obtained. The buffer used for the procedures may contain a protein modifier such as urea or guanidine hydrochloride, or a surfactant such as Triton X-100™, etc. When the protein of the present invention is secreted in the culture broth, the supernatant can be separated, after completion of the cultivation, from the bacteria or cell to collect the supernatant by a publicly known method.

The protein of the present invention contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein of the present invention thus obtained is in a free form, the protein can be converted into the salt by per se publicly known methods or modifications thereof. On the other hand, when the protein is obtained in the form of a salt, it can be converted into the free form or in the form of a different salt by per se publicly known methods or modifications thereof.

The protein produced by the recombinant can be treated, prior to or after the purification, with an appropriate protein-modifying enzyme so that the protein can be appropriately modified to partially remove the polypeptide. Examples of the protein-modifying enzyme include trypsin, chymotrypsin, arginyl endopeptidase, protein kinase, glycosidase and the like.

The presence of the thus produced protein of the present invention can be determined by an enzyme immunoassay or western blotting using a specific antibody.

The antibodies to the protein or partial peptide used in the present invention, or its salts may be any of polyclonal and monoclonal antibodies, as long as they are capable of recognizing the protein or partial peptide of the present invention, or its salts.

The antibodies to the protein or partial peptide used in the present invention, or its salts (hereinafter they are sometimes collectively referred to as the protein of the present invention in the description of the antibodies) can be produced by a publicly known method of producing an antibody or antiserum, using the protein of the present invention as an antigen.

### [Preparation of monoclonal antibody]

### (a) Preparation of monoclonal antibody-producing cells

The protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every about 2 to about 6 weeks and about 2 to about 10 times in total. Examples of the applicable warm-blooded animals are monkeys, rabbits, dogs; guinea pigs, mice, rats, sheep, goats and fowl, with the use of mice and rats being preferred.

In the preparation of monoclonal antibody-producing cells, a warm-blooded animal, e.g., mice, immunized with an antigen wherein the antibody titer is noted is selected, then spleen or lymph node is collected after 2 to 5 days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature, 256, 495, (1975)]. Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37 °C for 1 to 10 minutes, an efficient cell fusion can be carried out.

Various methods can be used for screening of monoclonal antibody-producing hybridomas. Examples of such: methods include a method which comprises adding the supernatant of a hybridoma to a solid phase (e.g., a microplate) adsorbed with the protein as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase, or the like.

The monoclonal antibody can be screened according to publicly known methods or their modifications. In general, the screening can be performed in a medium for animal cells supplemented with HAT (hypoxanthine, aminopterin and thymidine). Any screening and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like, can be used for the screening and growth medium. The culture is carried out generally at 20 to 40°C, preferably at 37°C, for about 5 days to about 3 weeks, preferably 1 to 2 weeks, normally in 5% CO₂. The antibody titer of the culture supernatant of a hybridoma can be determined as in the assay for the antibody titer in antisera described above.

### (b) Purification of monoclonal antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.]

### [Preparation of polyclonal antibody]

The polyclonal antibody of the present invention can be manufactured by publicly known methods or modifications thereof. For example, a warm-blooded animal is immunized with an immunogen (protein antigen) per se, or a complex of immunogen and a carrier protein is formed and a warm-blooded animal is immunized with the complex in a manner similar to the method described above for the manufacture of monoclonal antibodies. The product containing the antibody to the protein of the present invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks and about 3 to 10 times in total.

The polyclonal antibody can be collected from the blood, ascites, etc., preferably from blood of the warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described hereinabove.

The antisense nucleotide having a complementary or substantially complementary base sequence to the DNA encoding the protein or partial peptide of the present invention (hereinafter these DNAs are sometimes collectively referred to as the DNA of the present invention in the description of antisense nucleotide) can be any antisense nucleotide, so long as it possesses a base sequence complementary or substantially complementary base sequence to that of the DNA of the present invention and capable of suppressing expression of the DNA, but antisense DNA is preferred.

The base sequence substantially complementary to the DNA of the present invention may include, for example, a base sequence having at least about 70% homology, preferably at least about 80% homology, more preferably at least about 90% homology and most preferably at least about 95% homology, to the full-length base sequence or to the partial base sequence (i.e., complementary strand to the DNA of the present invention), and the like. Especially in the entire base sequence of the complementary strand to the DNA of the present invention, (a) in the case of antisense nucleotide directed to translation inhibition, an antisense nucleotide having at least about 70% homology, preferably at least about 80% homology, more preferably at least about 90% homology and most preferably at least about 95% homology, to the' complementary strand of the base sequence which encodes the N-terminal region of the protein of the present invention (e.g., the base sequence around the initiation codon, etc.), and (b) in the case of antisense nucleotide directed to RNA degradation by RNase H, an antisense nucleotide having at least about 70% homology, preferably at least about 80% homology, more preferably at least about 90% homology and most preferably at least about 95% homology, to the complementary strand of the entire base sequence of the DNA of the present invention containing intron are preferred, respectively.

Specific examples include an antisense polynucleotide containing the entire or part of a base sequence complementary or substantially complementary to a base sequence of DNA containing the base sequence represented by SEQ ID NO: 2 or SEQ ID NO: 3, preferably an antisense polynucleotide containing the entire or part of a base sequence complementary to a base sequence of DNA containing the base sequence represented by SEQ ID NO: 2 or SEQ ID NO: 3 (more preferably, an antisense polynucleotide containing the entire or part of a base sequence complementary to a base sequence of DNA containing the base sequence represented by SEQ ID NO: 2 or SEQ ID NO: 3), etc.

The antisense polynucleotide is generally constituted by bases of about 10 to about 40, preferably about 15 to about 30.

To prevent digestion with a hydrolase such as nuclease; etc., the phosphoric acid residue (phosphate) of each nucleotide that constitutes the antisense DNA may be substituted with chemically modified phosphoric acid residues, e.g., phosphorothioate, methyl phosphonate, phosphorodithionate, etc. The sugar (deoxyribose) in the respective nucleotides may also be substituted with a chemically modified sugar structure such as 2'-O-methylation, etc. and the base part (pyrimidine, purine) may undergo chemical modification, and can be any one so long as it hybridizes to a DNA having the base sequence represented by SEQ ID NO: 2. These antisense nucleotides may be synthesized using a publicly known DNA synthesizer, etc.

According to the present invention, the polynucleotide corresponding to a gene for the protein of the present invention that can inhibit replication or expression of the gene, e.g., an antisense polynucleotide (nucleic acid) can be designed and synthesized based on the base sequence information of the cloned or determined DNA encoding the protein. Such a polynucleotide (nucleic acid) is hybridizable to RNA of the protein gene of the present invention to inhibit the synthesis or function of said RNA or is capable of modulating or controlling the expression of the protein gene of the present invention via interaction with RNA associated with the protein of the present invention. Polynucleotides complementary to the selected sequences of RNA associated with the protein of the present invention and polynucleotides specifically hybridizable to RNA associated with the protein of the present invention are useful in modulating or controlling the in vivo and in vitro expression of the protein gene of the present invention, and are useful for the treatment or diagnosis of diseases, etc. The term "corresponding" is used to mean homologous to or complementary to a particular sequence of the nucleotide including the gene, base sequence or nucleic acid. The term "corresponding" between nucleotides, base sequences or nucleic acids and peptides (proteins) usually refer to amino acids of a peptide (protein) under the order derived from the sequence of nucleotides (nucleic acids) or their complements. In the protein genes, the 5' end hairpin loop, 5' end 6-base-pair repeats, 5' end untranslated region, polypeptide translation initiation codon, protein coding region, ORF translation termination codon, 3' end untranslated region, 3' end palindrome region, and 3' end hairpin loop, may be selected as preferred target regions, though any other region may be selected as a target in the protein genes.

The relationship between the targeted nucleic acids and the polynucleotides complementary to at least a part of the target region, specifically the relationship between the target nucleic acids and the polynucleotides hybridizable to the target region, can be denoted to be "antisense" to the polynucleotides in the said target region. Examples of the antisense (poly)nucleotides include polynucleotides containing 2-deoxy-D-ribose, polynucleotides containing D-ribose, any other type of polynucleotides which are N-glycosides of a purine or pyrimidine base, or other polymers containing non-nucleotide backbones (e.g., commercially available protein nucleic acids and synthetic sequence-specific nucleic acid polymers) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue; those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., α anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense (poly)nucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, increasing the cell permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense nucleic acid of the present invention may contain altered or modified sugars, bases or linkages. The antisense nucleic acid may also be provided in a specialized form such as liposomes, microspheres, or may be applied to gene therapy, or may be provided in combination with attached moieties. Such attached moieties include polycations such as. polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g.; cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense nucleic acid can be examined using the transformant of the present invention, the gene expression system of the present invention in vivo and in vitro, or the translation system of the protein in vivo and in vitro. The nucleic acid can be applied to cells by a variety of publicly known methods.

Hereinafter, the protein of the present invention, its partial peptides, or salts thereof (hereinafter sometimes merely referred to as the protein of the present invention), the DNA encoding the protein of the present invention or its partial peptides (hereinafter sometimes merely referred to as the DNA of the present invention), the antibodies to the protein of the present invention, its partial peptides, or salts thereof (hereinafter sometimes referred to as the antibodies of the present invention) and the antisense nucleotides to the DNA of the present invention (hereinafter sometimes merely referred to as the antisense nucleotides of the present invention) are specifically described for their applications.

The protein of the present invention can be utilized as a disease marker since expression of the protein increases in cancer tissues. That is, the protein is useful as a marker for early diagnosis in cancer tissues, judgment of severity in conditions, or predicted development of these diseases. Therefore, the pharmaceuticals comprising the antisense nucleotide of a gene encoding the protein of the present invention, the compound or its salt that inhibits the activities of the protein of the present invention, or the antibody to the protein of the present invention can be used as agents for the prevention/treatment of cancer, e.g., colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor, etc., apoptosis inducers, and the like.

### (1) Screening of drug candidate compounds for disease

Since the protein of the present invention increases its expression in cancer tissues to exhibit the apoptosis suppressing action, the compound or its salt that regulates (promotes or inhibits, preferably inhibits) the activities of the protein of the present invention can be used as agents for the prevention/treatment of colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor, etc. The protein of the present invention can also be used as an apoptosis action regulator, preferably an apoptosis inducer.

Thus, the protein of the present invention is useful as a reagent for screening the compound or its salt that regulates (promotes or inhibits, preferably inhibits) the activities of the protein of the present invention.

That is, the present invention provides a method of screening the compound or its salt that regulates (promotes or inhibits, preferably inhibits) the activities (e.g., the histone methyltransferase activity, etc:) of the protein of the present invention, which comprises using the protein of the present invention.

More specifically, the screening method includes, for example, a method of screening the compound or its salt that regulates (promotes or inhibits, preferably inhibits) the activities of the protein of the present invention, which comprises comparing (i) the histone methyltransferase activity of the protein of the present invention and (ii) the histone methyltransferase activity in a mixture of the protein of the present invention and a test compound.

In the screening method described above, for example, the histone methyltransferase activity in the cases of (i) and (ii) can be determined by methods per se publicly known, e.g., the method described in Nature, 406, 593-599, 2000, or its modifications.

Specifically, the radioactivities of histone H3 or polypeptide by transfer of the methyl group are assayed, respectively; (i) in the case where the protein of the present invention is reacted with S-adenosyl-L-methionine wherein the methyl group is radio-labeled and histone protein or a polypeptide having the N-terminal sequence of histone H3 and (ii) in the case where the protein of the present invention is reacted with S-adenosyl-L-methionine wherein the methyl group is radio-labeled and histone protein or a polypeptide having the N-terminal sequence of histone H3, in the presence of a test compound, whereby the compound or its salt that regulates (promotes or inhibits, preferably inhibits) the activities of the protein of the present invention is screened.

The reaction is carried out in an appropriate buffer. After the enzymatic reaction, the reaction product is separated, e.g., by SDS-PAGE, etc. and compared with the mobility of histone H3 or the like as a standard control to perform identification. Upon quantification, the radioactivity is assayed in accordance with publicly known methods using a scintillation counter, fluorography, etc.

S-Adenosyl-L-methionine with the radio-labeled methyl group, histone protein and the polypeptide having the N-terminal sequence of histone H3 may also be mixed with a test compound and the mixture is then reacted with the protein of the present invention. Alternatively, after S-adenosyl-L-methionine with the radio-labeled methyl group, histone protein and the polypeptide having the N-terminal sequence of histone H3 may be contacted with the protein of the present invention, a test compound may be added thereto.

Also, methylated (dimethylated/trimethylated) lysine residues are assayed using anti-histone H3 (dimethyl/trimethyl lysine 9) antibody, etc., respectively, (i') in the case where the protein of the present invention is reacted with S-adenosyl-L-methionine wherein the methyl group is radio-labeled and histone protein or a polypeptide having the N-terminal sequence of histone H3 and (ii') in the case where the protein of the present invention is reacted with S-adenosyl-L-methionine wherein the methyl group is radio-labeled and histone protein or a polypeptide having the N-terminal sequence of histone H3, in the presence of a test compound, whereby the compound or its salt that regulates (promotes or inhibits, preferably inhibits) the activities of the protein of the present invention is screened.

Furthermore, the reaction products obtained (i') in the case where the protein of the present invention is reacted with S-adenosyl-L-methionine wherein the methyl group is radio-labeled and histone protein or a polypeptide having the N-terminal sequence of histone H3 and (ii') in the case where the protein of the present invention is reacted with S-adenosyl-L-methionine wherein the methyl group is radio-labeled and histone protein or a polypeptide having the N-terminal sequence of histone H3, in the presence of a test compound, are appropriately purified and mass spectrometry is performed (using, e.g., TOF-MS, etc.). Thus, the compound or its salt that regulates (promotes or inhibits, preferably inhibits) the activities of the protein of the present invention is screened using as an indicator changes in molecular weight accompanied by methylation.

The protein of the present invention described above is preferably manufactured by culturing a transformant containing a DNA encoding the protein of the present invention. In addition, the reaction is carried out in a similar manner using a cell capable of expressing the protein of the present invention, and the radioactivity of histone H3 or polypeptide by transfer of the methyl group may also be assayed.

As the cells capable of producing the protein of the present invention, there are employed, e.g., the aforesaid host (transformant) transformed with a vector containing the DNA encoding the protein of the present invention. Preferably, animal cells such as COS7 cells, CHO cells, HEK293 cells, etc. are used as the host. For the screening, the transformant, in which the protein of the present invention has been expressed, e.g., by culturing through the procedure described above, is preferably employed. The same procedure for culturing the transformant of the present invention applies to the procedure for culturing the transformant with the protein of the present invention expressed.

Examples of the test compound include peptides, proteins, non-peptide compounds, synthetic compounds, fermentation products, cell extracts, plant extracts, animal tissue extracts, etc.

For example, when a test compound reduces the histone methyltransferase activity in the case (ii) as compared to the case (i) above by at least about 20%, preferably at least about 30% and more preferably at least about 50% can be selected as a compound capable of inhibiting the activity of the protein of the present invention; and when a test compound increaces the histone methyltransferase activity in the case (ii) as compared to the case (i) above by at least about 20%, preferably at least about 30% and more preferably at least about 50% can be selected as a compound capable of promoting the activity of the protein of the present invention.

The compound having the activity of inhibiting the activities of the protein of the present invention is useful as a safe and low toxic pharmaceutical to repress the physiological activities of the protein of the present invention, for example, as preventive/therapeutic agents for cancer such as colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor, etc., as apoptosis inducers, and the like.

The compound having the activity of promoting the activities of the protein of the present invention is useful as a safe and low toxic pharmaceutical to enhance the activities of the protein of the present invention.

The compound or its salt obtained using the screening method or screening kit of the present invention is a compound selected from, for example, peptides, proteins, non-peptide compounds, synthetic compounds, fermentation products, cell extracts, plant extracts, animal tissue extracts, plasma, etc. The salts of these compounds used are those given above as the salts of the peptide of the present invention.

In addition, since the gene encoding the protein of the present invention also increases its expression in cancer tissues, the compound or its salt that regulates the expression of the gene encoding the protein of the present invention can be used as preventive/therapeutic agents for cancer such as breast cancer, colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor, apoptosis inducers, etc.

Thus, the DNA of the protein of the present invention is useful as a reagent for screening the compound or its salt that regulates (promotes or inhibits, preferably inhibits) the expression of the gene encoding the protein of the present invention.

The screening method further includes a method of screening the inhibitor, which comprises comparing (iii) the case wherein a cell capable of producing the protein of the present invention is cultured and (iv) the case wherein a cell capable of producing the protein used in the present invention is cultured in the presence of a test compound.

In the screening method described above, the expression level of the gene described above (specifically, the level of the protein of the present invention or the level of mRNA encoding said protein) is determined in the cases (iii) and (iv), followed by comparing (iii) and (iv).

Examples of the test compound and the cell capable of producing the protein of the present invention are the same as those described above.

The level of the protein can be determined by publicly known methods, e.g., by measuring the aforesaid protein present in the cell extract, etc., using an antibody capable of recognizing the protein of the present invention, in accordance with methods such as western blot analysis, ELISA, etc., or modifications thereof.

The level of mRNA can be determined by publicly known methods, e.g., Northern blotting using as a probe SEQ ID NO: 2 or a nucleic acid containing a part thereof, PCR using as a primer SEQ ID NO: 2 or a nucleic acid containing a part thereof, or modifications thereof.

For example, when a test compound inhibits the expression level of the gene in the case (iv) described above by at least about 20%, preferably at least 30% and more preferably at least about 50%, as compared to the case (iii) above, the test compound can be selected to be a compound capable of inhibiting the expression of the gene encoding the protein of the present invention; and when a test compound increases the expression level of the gene in the case (iv) described above by at least about 20%, preferably at least 30% and more preferably at least about 50%, as compared to the case (iii) above, the test compound can be selected to be a compound capable of promoting the expression of the gene encoding the protein of the present invention.

The screening kit of the present invention comprises the protein or its partial peptide used in the present invention, or a salt thereof, or the cell capable of producing the protein or its partial peptide used in the present invention.

The compound or its salt obtained using the screening method or screening kit of the present invention is the test compound described above, e.g., the compound selected from peptides, proteins, non-peptide compounds, synthetic compounds, fermentation products, cell extracts, plant extracts, animal tissue extracts, plasma, etc., which is a compound or its salt that regulates the activities (e.g., the histone methyltransferase activity, etc.) of the protein of the present invention.

As salts of the compound, those as given for the salts of the protein of the present invention described above are used.

The compound or its salt that regulates (preferably inhibits) the activities of the protein of the present invention or the compound or its salt that regulates (preferably inhibits) the expression of the gene encoding the protein of the present invention are useful, respectively, as preventive/therapeutic agents for cancer such as colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor, etc., apoptosis inducers, and the like.

Where the compound or its salt obtained using the screening method or screening kit of the present invention is used as the preventive/therapeutic agent described above, the compound or its salt can be prepared into pharmaceutical preparations in a conventional manner.

For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or excipient conventionally used in the field of pharmaceutical preparations. Examples of the vehicle or excipient for tablets are lactose, starch, sucrose, magnesium stearate, etc.

As the composition for parenteral administration, injectable preparations, suppositories, etc. are used. The injectable preparations include dosage forms such as intravenous injection, subcutaneous injection, intracutaneous injection, intramuscular injection, drip infusions, intra-articular injection, etc. These injectable preparations may be prepared by methods publicly known, for example, by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate dissolution aid such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a dissolution aid such as benzyl benzoate, benzyl alcohol, etc. The injection thus prepared is preferably filled in an appropriate ampoule. The suppository used for rectal administration may be prepared by blending the aforesaid antibody or its salt with conventional bases for suppositories.

Advantageously, the pharmaceutical compositions for oral or parenteral use described, above are prepared into pharmaceutical preparations with a unit. dose suited to fit a dose of the active ingredients. Such unit dose preparations include, for example, tablets, pills, capsules, injections (ampoules), suppositories, etc. The amount of the aforesaid antibody contained is generally 5 to 500 mg per dosage unit form; it is preferred that the aforesaid antibody is contained in about 5 to about 100 mg especially in the form of injection, and in 10 to 250 mg for the other forms.

Each composition described above may further contain other active components unless formulation with the antibody described above causes any adverse interaction.

Since the pharmaceutical preparations thus obtained are safe and low toxic, they can be administered to human or warm-blooded animal (e.g., mouse, rat, rabbit, sheep, swine, bovine, horse, fowl, cat, dog, monkey, chimpanzee, etc.) orally or parenterally.

The dose of the compound or its salt may vary depending upon its action, target disease, subject to be administered, route of administration, etc. When the compound or its salt that regulates (preferably inhibits) the activities of the protein of the present invention is orally administered for the treatment of, e.g., breast cancer, the compound or its salt is generally administered to an adult (as 60 kg body weight) in a daily dose of about 0.1 to about 100 mg, preferably about 1.0 to about 50 mg and more preferably about 1.0 to about 20 mg. In parenteral administration, a single dose of the aforesaid compound or its salt may vary depending upon subject to be administered, target disease, etc. When the compound or its salt that regulates (preferably inhibits) the activities of the protein of the present invention is administered to an adult (as 60 kg body weight) in the form of an injectable preparation for the treatment of, e.g., breast cancer, it is advantageous to administer the compound or its salt intravenously in a daily dose of about 0.01 to about 30 mg, preferably about 0.1 to about 20 mg, and more preferably about 0.1 to about 10 mg. For other animal species, the corresponding dose as converted per 60 kg weight can be administered.

Furthermore, the compound described above can be used in combination with the existing anticancer agents [e.g., alkylating agents (e.g., cyclophosphamide, ifosfamide, nimustine, ranimustine, carboquone, etc.), antimetabolites (e.g., methotrexate, 5-fluorouracil, tegafur, carmofur, UFT, doxifluridine, cytarabine, enocitabine, mercaptopurine, mercaptopurine riboside, thioguanine, etc.), anticancer antibiotics (e.g., mitomycin, adriamycin, daunorubicin, epirubicin, pirarubicin, idarubicin, bleomycin, peplomycin, actinomycin, etc.), plant-derived anticancer agents (e.g., vincristine, vinblastine, vindesine, etoposide, camptothecine, irinotecan, etc.), cisplatin, carboplatin, nedaplatin, paclitaxel, docetaxel, estramustine, etc.]. In this case, the time for administration is not limited but they may be given to the subject to be administered, simultaneously or with time difference. The dose can be appropriately chosen based on the standard dose clinically used. Also, a formulation ratio of the aforesaid compound to the anticancer agent can be adequately chosen depending upon subject to be administered, route for administration, disease of interest, conditions, combinations, etc.

### (2) Quantification for the protein of the present invention, it partial peptide or salts thereof

The antibody to the protein of the present invention (hereinafter sometimes merely referred to as the antibody of the present invention) is capable of specifically recognizing the protein of the present invention, and thus can be used for quantification of the protein of the present invention in a test sample fluid, in particular, for quantification by sandwich immunoassay; etc.

That is, the present invention provides:
(i) a method for quantification of the protein of the present invention in a test sample fluid, which comprises competitively reacting the antibody of the present invention, a test sample fluid and a labeled form of the protein of the present invention, and measuring the ratio of the labeled form of the protein of the present invention bound to said antibody; and,
(ii) a method for quantification of the protein of the present invention in a test sample fluid, which comprises reacting a test sample fluid simultaneously or continuously with the antibody of the present invention immobilized on a carrier and another labeled antibody of the present invention, and then measuring the activity of the labeling agent on the insoluble carrier.

In the quantification method (ii) described above, it is preferred that one antibody is capable of recognizing the N-terminal region of the protein of the present invention, while another antibody is capable of reacting with the C-terminal region of the protein of the present invention.

The monoclonal antibody to the protein of the present invention (hereinafter sometimes referred to as the monoclonal antibody of the present invention) can be used to quantify the protein of the present invention. In addition, the protein can be detected by means of a tissue staining as well. For these purposes, the antibody molecule per se may be used or F(ab')₂, Fab' or Fab fractions of the antibody molecule may also be used.

The method for quantification of the protein of the present invention using the antibody of the present invention is not particularly limited. Any quantification method can be used, so long as the amount of antibody, antigen or antibody-antigen complex corresponding to the amount of antigen (e.g., the amount of the protein) in a test sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is particularly preferred to use the sandwich method described hereinafter.

Examples of the labeling agent used in the assay method using the labeling substance are radioisotopes, enzymes, fluorescent substances, luminescent substances, and the like. As the radioisotopes, there are used, e.g., [¹²⁵I], [¹³¹I], [³H], [¹⁴C], etc. The enzymes described above are preferably enzymes, which are stable and have a high specific activity, and include, e.g., beta-galactosidase, beta-glucosidase, an alkaline phosphatase, a peroxidase, malate dehydrogenase, etc. As the fluorescent substances, there are used, e.g., fluorescamine, fluorescein isothiocyanate, etc. As the luminescent substances described above there are used, e.g., luminol, a luminol derivative, luciferin, lucigenin, etc. Furthermore, the biotin-avidin system may be used as well for binding of an antibody or antigen to a labeling agent.

For immobilization of the antigen or antibody, physical adsorption may be used. Chemical binding techniques conventionally used for insolubilization or immobilization of proteins, enzymes, etc. may also be used. For carriers, there are used, e.g., insoluble polysaccharides such as agarose, dextran, cellulose, etc.; synthetic resin such as polystyrene, polyacrylamide, silicon, etc., and glass or the like.

In the sandwich method, the immobilized monoclonal antibody of the present invention is reacted with a test sample fluid (primary reaction), then with a labeled form of another monoclonal antibody of the present invention (secondary reaction), and the activity of the label on the immobilizing carrier is measured, whereby the amount of the protein of the present invention in the test sample fluid can be quantified. The order of the primary and secondary reactions may be reversed, and the reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity.

In the methods of assaying the protein of the present invention by the sandwich method, antibodies that bind to different sites of the protein of the present invention are preferably used as the monoclonal antibodies of the present invention used for the primary and secondary reactions. That is, in the antibodies used for the primary and secondary reactions are, for example, when the antibody used in the secondary reaction recognizes the C-terminal region of the protein of the present invention, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, the competitive method, the immunometric method, nephrometry, etc.

In the competitive method, antigen in a test sample fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test sample fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test sample fluid and immobilized antigen are competitively reacted with a definite amount of labeled antibody, the immobilized phase is separated from the liquid phase, or antigen in a test sample fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test sample fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying these immunological methods to the measurement methods of the present invention, any particular conditions or procedures are not required. Systems for measuring the protein of the present invention or its salts are constructed by adding the usual technical consideration in the art to the conventional conditions and procedures. For the details of these general technical means, reference can be made to the following reviews and texts.

For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978),' Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing).

As described above, the protein of the present invention can be quantified with high sensitivity, using the antibody of the present invention.

Furthermore, when an increased level or decreased level of the protein of the present invention is detected by quantifying the level of the protein of the present invention using the antibody of the present invention, it can be diagnosed that one suffers' from cancer such as colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor, etc., or it is highly likely to suffer from these disease in the future.

Moreover, the antibody of the present invention can be used to detect the protein of the present invention, which is present in a test sample fluid such as a body fluid, a tissue, etc. The antibody can also be used to prepare an antibody column for purification of the protein of the present invention, detect the protein of the present invention in each fraction upon purification, analyze the behavior of the protein of the present invention in the cells under investigation; etc.

### (3) Gene diagnostic agent

By using the DNA of the present invention, e.g., as a probe, an abnormality (gene abnormality) of the DNA or mRNA encoding the protein of the present invention or its partial peptide in human or warm-blooded animal (e.g., rat, mouse, guinea pig, rabbit, fowl, sheep, swine, bovine, horse, cat, dog, monkey, chimpanzee, etc.) can be detected. Therefore, the DNA of the present invention is useful as a gene diagnostic agent for detecting damages to the DNA or mRNA, its mutation, or decreased expression, increase in or overexpression of the DNA or mRNA, and so on.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), etc.

When overexpression or decrease is detected by, e.g., the Northern hybridization or DNA mutation is detected by the PCR-SSCP assay, it can be diagnosed that it is highly likely to suffer from cancer such as colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor, etc.

### (4) Pharmaceutical comprising an antisense nucleotide

The antisense nucleotide of the present invention that binds to the DNA of the present invention complementarily to inhibit expression of the DNA is low toxic and can suppress the functions (e.g., the histone methyltransferase activity) of the protein of the present invention or the DNA of the present invention in vivo. Thus, the antisense nucleotide can be used as a preventive/therapeutic agent for cancer such as colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor, etc., an apoptosis inducer, and the like.

Where the antisense nucleotide described above is used as the preventive/therapeutic agent described above, it can be prepared into pharmaceutical preparations by per se publicly known methods, which are provided for administration.

For example, when the antisense nucleotide is used, the antisense nucleotide alone is administered directly, or the antisense nucleotide is inserted into an appropriate vector such as retrovirus vector, adenovirus vector, adenovirus-associated virus vector, etc., followed by treating in a conventional manner. The antisense nucleotide may then be administered orally or parenterally to human or non-human mammal (e.g., rat, rabbit, sheep, swine, bovine, cat, dog, monkey, etc.) in a conventional manner. The antisense nucleotide may also be administered as it stands, or may be prepared in pharmaceutical preparations together with a physiologically acceptable carrier to assist its uptake, which are then administered by gene gun or through a catheter such as a catheter with a hydrogel. Alternatively, the antisense nucleotide may be prepared into an aerosol, which is topically administered into the trachea as an inhaler.

Further for the purposes of improved pharmacokinetics, prolonged half life and improved efficiency in intracellular uptake, the antisense nucleotide described above may also be prepared into pharmaceutical preparations (injections), solely or together with carriers such as liposome, etc., which may be administered intravenously, subcutaneously or intra-articularly, or to the cancer lesion site, etc.

The dose of the antisense nucleotide may vary depending on target disease, subject to be administered, route for administration, etc. When the antisense nucleotide of the present invention is administered for the treatment of, e.g., breast cancer, the antisense nucleotide is generally administered to an adult (body weight of 60 kg) in a daily dose of about 0.1 to about 100 mg.

In addition, the antisense nucleotide may also be used as an oligonucleotide probe for diagnosis to examine the presence of the DNA of the present invention in tissues or cells and states of its expression.

As in the antisense nucleotide described above, double-stranded RNA containing a part of RNA encoding the protein of the present invention, ribozyme containing a part of RNA encoding the protein of the present invention, etc. can also suppress the expression of the gene of the present invention and can suppress the in vivo function of the protein used in the present invention or the DNA used in the present invention. Therefore, they can be used as preventive/therapeutic agents for cancer such as colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor, etc., apoptosis inducers, and the like.

The double-stranded RNA can be designed based on a sequence of the polynucleotide of the present invention and manufactured by modifications of publicly known methods (e.g., Nature, 411, 494, 2001).

The ribozyme can be designed based on a sequence of the polynucleotide of the present invention and manufactured by modifications of publicly known methods (e.g., TRENDS in Molecular Medicine, 7, 221, 2001). For example, the ribozyme can be manufactured by ligating a publicly known ribozyme to a part of the RNA encoding the protein of the present invention. A part of the RNA encoding the protein of the present invention includes an adjacent portion to a cleavage site on, the RNA of the present invention, which may be cleaved by a publicly known ribozyme (RNA fragment).

Where the double-stranded RNA or ribozyme described above can be used as the preventive/therapeutic agent described above, the ribozyme can be prepared into pharmaceutical preparations, which are provided for administration, as in the antisense polynucleotide.

### (5) Pharmaceutical comprising the antibody of the present invention

The antibody having the action of neutralizing the activities of the protein of the present invention can be used as preventive/therapeutic agent for cancer such as colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor, etc., apoptosis inducers, and the like.

The preventive/therapeutic agent described above comprising the antibody of the present invention is low toxic and can be administered to human or mammal (e.g., rat, rabbit, sheep, swine, bovine, cat, dog, monkey, etc.) orally or parenterally (e.g., intravenous injection). The dose may vary depending upon subject to be administered, target disease, conditions, route of administration, etc. In general, when it is used for the treatment/prevention of, e.g., breast cancer of an adult, it is advantageous to administer the antibody of the present invention intravenously in a single dose of about 0.01 to about 20 mg/kg body weight, preferably about 0.1 to about 10 mg/kg body weight, more preferably about 0.1 to about 5 mg/kg body weight, in about 1 to about 5 times per day, preferably in about 1 to about 3 times per day. In other parenteral administration and oral administration, the antibody can be administered in a dose corresponding to the above dose. When the condition is especially severe, the dose may be increased accordingly to the condition.

The antibody of the present invention may be administered in itself or as an appropriate pharmaceutical composition. The pharmaceutical composition used for the administration described above contains the aforesaid antibody or its salts and a pharmacologically acceptable carrier, diluent or excipient. Such a composition is provided in the form of pharmaceutical preparations suitable for oral or parenteral (e.g., intravenous injection) administration.

Each composition described above may further contain other active components unless formulation with the antibody described above causes any adverse interaction.

### (6) Regarding "the preventive/therapeutic agent for cancer comprising the compound or its salt having the action of regulating the histone methyltransferase activity" and "the preventive/therapeutic agent for cancer comprising the compound or its salt having the action of regulating the expression of histone methyltransferase" of the present invention

The "compound having the action of regulating the histone methyltransferase activity" may be any compound, so long as the compound has the action of regulating the histone methyltransferase activity. The compound can be used as preventive/therapeutic agent for cancer such as colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor, etc., an apoptosis inducer, and the like.

The "compound having the action of regulating the expression of histone methyltransferase" may be any compound, so long as the compound has the action of regulating the expression of histone methyltransferase. The compound can be used as preventive/therapeutic agent for cancer such as colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor, etc., an apoptosis inducer, and the like.

The preventive/therapeutic agent is manufactured as described above.

### (7) DNA transgenic animal

The present invention provides a non-human mammal bearing DNA encoding the protein of the present invention, which is exogenous (hereinafter abbreviated as the exogenous DNA of the present invention) or its variant DNA (sometimes simply referred to as the exogenous variant DNA of the present invention).

That is, the present invention provides:
(1) A non-human mammal bearing the exogenous DNA of the present invention or its variant DNA;
(2) The mammal according to (1), wherein the non-human mammal is a rodent;
(3) The mammal according to (2), wherein the rodent is mouse or rat; and,
(4) A recombinant vector containing the exogenous DNA of the present invention or its variant DNA and capable of expressing in a mammal.

The non-human mammal bearing the exogenous DNA of the present invention or its variant DNA (hereinafter simply referred to as the DNA transgenic animal of the present invention) can be created by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, it is possible to transfect the exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to prepare the DNA transgenic animal of the present invention.

Examples of the non-human mammal that can be used include bovine, swine, sheep, goat, rabbits, dogs, cats, guinea pigs, hamsters, mice, rats, etc. Above all, preferred are rodents, especially mice (e.g., C57B1/6 strain, DBA2 strain, etc. for a pure line and for a cross line, B6C3F₁ strain, BDF₁ strain B6D2F₁ strain, BALB/c strain, ICR strain, etc.), rats (Wistar, SD, etc.) or the like, since they are relatively short in ontogeny and life cycle from a standpoint of creating model animals for human disease.

"Mammals" in a recombinant vector that can be expressed in the mammals include the aforesaid non-human mammals and human.

The exogenous DNA of the present invention refers to the DNA of the present invention that is once isolated and extracted from mammals, not the DNA of the present invention inherently possessed by the non-human mammals.

The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean DNA that expresses the abnormal protein of the present invention and exemplified by the DNA that expresses a protein for suppressing the function of the normal protein of the present invention.

The abnormal DNA is intended to mean DNA that expresses the abnormal protein of the present invention and exemplified by the DNA that expresses a protein for suppressing the function of the normal protein of the present invention.

The exogenous DNA of the present invention may be any one of those derived from a mammal of the same species as, or a different species from, the mammal as the target animal. In transfecting the DNA of the present invention, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the protein of the present invention, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression include (1) promoters for DNA derived from viruses (e.g., simian virus, cytomegalovirus, Moloney leukemia virus, JC virus, breast cancer virus, poliovirus, etc.), and promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), protein chain elongation factor 1α(EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human protein elongation factor 1α(EF-1 α) promoters, human and chicken β actin promoters, etc., which are capable of high expression in the whole body are preferred.

Preferably, the vectors described above have a sequence that terminates the transcription of the desired messenger RNA in the DNA transgenic animal (generally termed a terminator); for example, a sequence of each DNA derived from viruses and various mammals, and SV40 terminator of the simian virus and the like are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal protein of the present invention can be obtained using as a starting material the entire genomic DNA or its portion of liver, kidney, thyroid cell or fibroblast origin from human or various mammals (e.g., rabbits, dogs, cats, guinea pigs, hamsters, rats, mice, etc.) or of various commercially available genomic DNA libraries, or using cDNA prepared by a publicly known method from RNA of liver, kidney, thyroid cell or fibroblast origin as a starting material. Also, an exogenous abnormal DNA can produce the translational region through variation of the translational region of normal protein obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the' germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by crossing.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the DNA of the present invention is excessively present in all of the germinal cells and somatic cells thereof. The offspring of the animal that inherits the exogenous DNA of the present invention have excessively the DNA of the present invention in all of the germinal cells and somatic cells thereof.

It is possible to obtain homozygotic animals having the transfected DNA in both homologous chromosomes and breed male and female of the animal so that all the progeny have this DNA in excess.

In a non-human mammal bearing the normal DNA of the present invention, the normal DNA of the present invention has expressed at a high level, and may eventually develop hyperfunction in the function of the protein of the present invention by promoting the function of endogenous normal DNA. Therefore, the animal can be utilized as a pathologic model animal for such a disease. For example, using the normal DNA transgenic animal of the present invention, it is possible to elucidate the mechanism of hyperfunction in the function of the protein of the present invention and the pathological mechanism of the disease associated with the protein of the present invention and to investigate how to treat these diseases.

Furthermore, since a mammal transfected with the exogenous normal DNA of the present invention exhibits an increasing symptom of the protein of the present invention liberated, the animal can be utilized in a screening test of preventive/therapeutic agents for diseases associated with the protein of the present invention, for example, cancer such as colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor, etc., or an apoptosis inducer.

On the other hand; a non-human mammal having the exogenous abnormal DNA of the present invention can be passaged under normal breeding conditions as the DNA-bearing animal by confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfection of the abnormal DNA of the present invention at the fertilized egg cell stage is preserved to be present in all of the germinal and somatic cells of the target mammal. The fact that the abnormal DNA of the present invention is present in the germinal cells of the animal after DNA transfection means that all of the offspring of the prepared animal have the abnormal DNA of the present invention in all of the germinal and somatic cells. Such an offspring that passaged the exogenous DNA of the present invention will have the abnormal DNA of the present invention in all of the germinal and somatic cells. A homozygous animal having the introduced DNA on both of homologous chromosomes can be acquired, and by crossing these male and female animals, all the offspring can be bred to retain the DNA.

In a non-human mammal bearing the abnormal DNA of the present invention, the abnormal DNA of the present invention has expressed to a high level, and may eventually develop the function inactive type inadaptability to the protein of the present invention by inhibiting the functions of endogenous normal DNA. Therefore, the animal can be utilized as a pathologic model animal for such a disease. For example, using the abnormal DNA transgenic animal of the present invention, it is possible to elucidate the mechanism of the function inactive type inadaptability to the protein of the present invention and the pathological mechanism of the disease associated with the protein of the present invention and to investigate how to treat the disease.

More specifically, the transgenic animal of the present invention in which the abnormal DNA of the present invention is expressed at a high level is expected to serve as an experimental model to elucidate the mechanism of the functional inhibition (dominant negative effect) of a normal protein by the abnormal protein of the present invention in the function inactive type inadaptability of the protein of the present invention.

In a mammal transferred with the abnormal exogenous DNA of the present invention, a liberated form of the protein of the present invention increases in the animal. Thus, the animal is also expected to be utilized in a screening test of preventive/therapeutic agents for the function inactive type inadaptability of the protein of the present invention, for example, cancer such as colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor, etc., or an apoptosis inducer.

Other potential applications of two kinds of the DNA transgenic animals of the present invention described above further include:
(1) Use as a cell source for tissue culture;
(2) Elucidation of the relation to a peptide that is specifically expressed or activated by the protein of the present invention, by direct analysis of DNA or RNA in tissues of the DNA transgenic animal of the present invention or by analysis of the peptide tissues expressed by the DNA;
(3) Research on the function of cells derived from tissues that are usually cultured only with difficulty; using cells in tissues bearing the DNA cultured by a standard tissue culture technique;
(4) Screening a drug that enhances the functions of cells using the cells described in (3) above; and,
(5) Isolation and purification of the variant protein of the present invention and preparation of an antibody thereto; etc.
   Furthermore, clinical conditions of a disease associated wit the protein of the present invention, including the function inactive type inadaptability to the protein of the present invention can be determined by using the DNA transgenic animal of the present invention. Also, pathological findings on each organ in a disease model associated with the protein of the present invention can be obtained in more detail, leading to the development of a new method for treatment as well as the research and therapy of any secondary diseases associated with the disease.
   It is also possible to obtain a free DNA-transfected cell by withdrawing each organ from the DNA transgenic animal of the present invention, mincing the organ and degrading with a proteinase such as trypsin, etc., followed by establishing the line of culturing or cultured cells. Furthermore, the DNA transgenic animal of the present invention can serve to identify cells capable of producing the protein of the present invention, and to study in association with apoptosis, differentiation or propagation or on the mechanism of signal transduction in these properties to inspect any abnormality therein. Thus, the DNA transgenic animal can provide an effective research material for the protein of the present invention and for investigation of the function and effect thereof.
   To develop a drug for the treatment of diseases associated with the protein of the present invention, including the function inactive type inadaptability to the protein of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the protein of the present invention, using the DNA transgenic animal of the present invention or a vector capable of expressing the exogenous DNA of the present invention.

### (8) Knockout animal

The present invention provides a non-human mammal embryonic stem cell bearing the DNA of the present invention inactivated and a non-human mammal deficient in expressing the DNA of the present invention.

Thus, the present invention provides:
(1) A non-human mammal embryonic stem cell in which the DNA of the present invention is inactivated;
(2) The embryonic stem cell according to (1), wherein the DNA is inactivated by transfecting a reporter gene (e.g., β-galactosidase gene derived from Escherichia coli);
(3) The embryonic stem cell according to (1), which is resistant to neomycin;
(4) The embryonic stem cell according to (1), wherein the non-human mammal is a rodent;
(5) The embryonic stem cell according to (4), wherein the rodent is mouse;
(6) A non-human mammal deficient in expressing the DNA of the present invention, wherein the DNA is inactivated;
(7) The non-human mammal according to (5), wherein the DNA is inactivated by inserting a reporter gene (e.g., β-galactosidase derived from Escherichia coli) therein and the reporter gene is capable of being expressed under control of a promoter for the DNA of the present invention;
(8) The non-human mammal according to (6), which is a rodent;
(9) The non-human mammal according to (8), wherein the rodent is mouse; and,
(10) A method of screening a compound that promotes or inhibits (preferably inhibits) the promoter activity to the DNA of the present invention, which comprises administering a test compound to the mammal of (7) and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the protein of the present invention (hereinafter sometimes referred to as the knockout DNA of the present invention) by substantially inactivating the activities of the protein of the present invention encoded by the DNA (hereinafter merely referred to as ES cell).

As the non-human mammal; the same examples as described above apply.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the target animal by, e.g., homologous recombination, a DNA sequence that terminates gene transcription (e.g., polyA additional signal, etc.) in the intron between exons, thus inhibiting the synthesis of complete messenger RNA and eventually destroying the gene (hereinafter simply referred to as a targeting vector). The thus-obtained ES cells to the southern hybridization analysis with a DNA sequence on or near the DNA of the present invention as a probe, or to PCR analysis with a DNA sequence on the targeting vector and another DNA sequence near the DNA of the present invention which is not included in the targeting vector as primers, to screen the knockout ES cell of the present invention.

The parent ES cells to inactivate the DNA of the present invention by homologous recombination, etc. may be of a strain already established as described above, or may originally be established in accordance with a modification of the known method by Evans and Kaufman described above. For example, in the case of mouse ES cells, currently it is common practice to use ES cells of the 129 strain. However, since their immunological background is obscure, the C57BL/6 mouse or the BDF₁ mouse (F₁ hybrid between C57BL/6 and DBA/2), wherein the low ovum availability per C57BL/6 in the C57BL/6 mouse has been improved by crossing with DBA/2, may be preferably used, instead of obtaining a pure line of ES. cells with the clear immunological genetic background and for other purposes. The BDF₁ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. In the present invention, embryos are preferably collected at the 8-cell stage, after culturing until the blastocyte stage, the embryos are used to efficiently obtain a large number of early stage embryos.

Although the ES cells used may be of either sex, male ES cells are generally more convenient for generation of a germ cell line chimera. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about 10⁶ cells; therefore, the first selection of ES cells at the early stage of culture can be based on sex identification, and male cells can be selected early, which saves a significant amount of time at the early stage of culture.

Also, second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g:, in a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably 5% carbon dioxide and 95% air, or 5% oxygen, 5% carbon dioxide and 90% air) in the presence of LIF (1 to 10000 U/ml) on appropriate feeder cells such as STO fibroblasts, treated with a trypsin/EDTA solution (normally 0.001 to 0.5% trypsin/0.1 to about 5 mM EDTA, preferably about 0.1% trypsin/1 mM EDTA) at the time of passage to obtain separate single cells, which are then plated on freshly prepared feeder cells. This passage is normally conducted every 1 to 3 days; it is desirable that cells be observed at the passage and cells found to be morphologically abnormal in culture, if any, be abandoned.

Where ES cells are allowed to reach a high density in mono-layers or to form cell aggregates in suspension under appropriate conditions, it is possible to differentiate the ES cells to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the present invention, which are obtained from the differentiated ES cells of the present invention, are useful for studying the function of the protein of the present invention cytologically.

The non-human mammal deficient in expression of the DNA of the present invention can be identified from a normal animal by measuring the mRNA level in the subject animal by a publicly known method, and indirectly comparing the degrees of expression.

As the non-human mammal, the same examples supra apply.

With respect to the non-human mammal deficient in expression of the DNA of the present invention, the DNA of the present invention can be made knockout by transfecting a targeting vector, prepared as described above, to mouse embryonic stem cells or mouse oocytes, and conducting homologous recombination in which a targeting vector DNA sequence, wherein the DNA of the present invention is inactivated by the transfection, is replaced with the DNA of the. present invention on a chromosome of a mouse embryonic stem cell or mouse embryo.

The knockout cells with the disrupted DNA of the present invention can be identified by the southern hybridization analysis using as a probe a DNA fragment on or near the DNA of the present invention, or by the PCR analysis using as primers a DNA sequence on the targeting vector and another DNA sequence at the proximal region of other than the DNA of the present invention derived from mouse used in the targeting vector. When non-human mammal stem cells are used, a cell line wherein the DNA of the present invention is inactivated by homologous recombination is cloned; the resulting clones are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the protein of the present invention. The individuals deficient in homozygous expression of the protein of the present invention can be obtained from offspring of the intercross between those deficient in heterozygous expression of the protein of the present invention.

When an oocyte is used, a DNA solution may be injected, e.g., into the prenucleus by microinjection thereby to obtain a transgenic non-human mammal having' a' targeting vector introduced in its chromosome. From such transgenic non-human mammals, those having a mutation at the locus of the DNA of the present invention can be obtained by selection based on homologous recombination.

As described above, the individuals in which the DNA of the present invention is rendered knockout permit passage rearing under ordinary rearing conditions, after the individuals obtained by their crossing have proven to have been knockout.

Furthermore, the genital system may be obtained and retained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell, in which the DNA of the present invention is inactivated, is very useful for preparing a non-human mammal deficient in expression of the DNA of the present invention.

Since the non-human mammal, in which the DNA of the present invention is inactivated, lacks various biological activities derived from the protein of the present invention, such an animal can be a disease model suspected of inactivated biological activities of the protein of the present invention and thus, offers an effective study to investigate the causes for and therapy for these diseases.

### (8a) Method of screening a compound having a therapeutic/preventive effect on diseases caused by deficiency, damages, etc. of the DNA of the present invention

The non-human mammal deficient in expression of the DNA of the present invention can be employed for screening of a compound having a therapeutic/preventive effect on diseases caused by deficiency, damages, etc. of the DNA of the present invention.

That is, the present invention provides a method of screening a compound having a therapeutic/preventive effect on diseases caused by deficiency, damages, etc. of the DNA of the present invention, which comprises administering a test compound to a non-human mammal deficient in expression of the DNA of the present invention and, observing and measuring a change occurred in the animal.

As the non-human mammal deficient in expression of the DNA of the present invention, which can be employed for the screening method, the same examples as given hereinabove apply.

Examples of the test compound include peptides, proteins, non-peptide compounds, synthetic compounds, fermentation products, cell extracts, plant extracts, animal tissue extracts, blood plasma, etc. These compounds may be novel compounds or publicly known compounds.

Specifically, the non-human mammal deficient in expression of the DNA of the present invention is treated with a test compound, comparison is made with an intact animal for control and a change in each organ, tissue, disease conditions, etc. of the animal is used as an indicator to assess the therapeutic/preventive effects of the test compound.

For treating an animal to be tested with a test compound, for example, oral administration, intravenous injection, etc. are applied, and the treatment can be appropriately selected depending on conditions of the test animal, properties of the test compound, etc. Furthermore, a dose of the test compound to be administered can be appropriately selected depending on the administration route, nature of the test compound, etc.

For screening of the compound having a therapeutic/preventive effect on cancer such as colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor, etc., a test compound is given to the non-human mammal deficient in expression of the DNA of the present invention and differences in degree of cancer onset or differences in degree of cancer cure from the group added with no test compound are observed in the tissues described above with passage of time.

In the screening method, when a test compound is given to the test animal and the disease conditions in the test animal are improved by at least about 10%, preferably at least about 30% and more preferably at least about 50%, the test compound can be selected as a compound having the therapeutic/preventive effect on the disease described above.

The compound obtained using the above screening method is a compound selected from the test compounds described above and exhibits therapeutic/preventive effects on diseases caused by deficiencies, damages, etc. of the protein of the present invention. Therefore, the compound can be employed as a safe and low toxic drug for the prevention/treatment of the diseases. Furthermore, compounds derived from the compound obtained by the screening described above may also be used as well.

The compound obtained by the screening method above may form salts, and may be used in the form of salts with physiologically acceptable acids (e.g., inorganic acids, organic acids, etc.) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

A pharmaceutical comprising the compound obtained by the above screening method or salts thereof can be manufactured in a manner similar to the method for preparing the pharmaceutical comprising the protein of the present invention described hereinabove.

Since the pharmaceutical preparation thus obtained is safe and low toxic, it can be administered to human or non-human mammal (e.g., rat, mouse, guinea pig, rabbit, sheep, swine, bovine, horse, cat, dog, monkey, etc.).

The dose of the compound or its salt may vary depending upon target disease, subject to be administered; route of administration, etc. For example, when the compound is orally administered to an adult (as 60 kg body weight), the compound is generally administered to the patient with, e.g.; breast cancer in a daily dose of about 0.1 to about 100 mg, preferably about 1.0 to about 50 mg and, more preferably about 1.0 to about 20 mg. In parenteral administration, a single dose of said compound or its salt may vary depending upon target subject, target disease, etc. When the compound or its salt is administered to an adult (as 60 kg) with breast cancer in the form of an injectable preparation, it is advantageous to administer the compound intravenously to the patient in a daily dose of about 0.01 to about 30 mg, preferably about 0.1 to about 20 mg, and more preferably about 0.1 to about 10 mg. For other animal species, the corresponding dose as converted per 60 kg weight can be administered.

### (8b) Method of screening a compound that promotes or inhibits the activities of a promoter to the DNA of the present invention

The present invention provides a method of screening a compound or its salts that promote or inhibit the activity of a promoter to the DNA of the present invention, which comprises administering a test compound to a non-human mammal deficient in expression of the DNA of the present invention and detecting the expression of the reporter gene.

In the screening method described above, an animal in which the DNA of the present invention is inactivated by transfecting a reporter gene and the reporter gene is expressed under control of a promoter to the DNA of the present invention is used as the non-human mammal deficient in expression of the DNA of the present invention, which is selected from the aforesaid non-human mammals deficient in expression of the DNA of the present invention.

The same examples of the test compound apply to specific compounds used for the screening.

As the reporter gene, the same specific examples apply to this screening method. Preferably, there are used β-galactosidase (lacZ), soluble. alkaline phosphatase gene, luciferase gene and the like.

Since the reporter gene is present under control of a promoter to the DNA of the present invention in the non-human mammal deficient in expression of the DNA of the present invention wherein the DNA of the present invention is substituted with the reporter gene, the activities of the promoter can be detected by tracing the expression of a substance encoded by the reporter gene.

When a part of the DNA region encoding the protein of the present invention is substituted with, e.g., β-galactosidase gene (lacZ) derived from Escherichia coli, β-galactosidase is expressed in a tissue where the protein of the present invention should originally be expressed, instead of the protein of the present invention. Thus, the state of expression of the protein of the present invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening method described above are compounds that are selected from the test compounds described above and that promote or inhibit the promoter activities to the DNA of the present invention.

The compound obtained by the screening method above may form salts, and may be used in the form of salts with physiologically acceptable acids (e.g., inorganic acids, etc.) or bases (e.g., organic acids, etc.) or the like, especially in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salt that promotes or inhibits the promoter activities to the DNA of the present invention can regulate the expression of the protein of the present invention and can regulate the functions of the aforesaid protein. Thus, the compound or its salt is useful as preventive/therapeutic agents for cancer such as colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor, etc., apoptosis inducers, and the like.

In addition, compounds derived from the compound obtained by the screening described above may also be used as well.

A pharmaceutical comprising the compound obtained by the above screening method or salts thereof can be manufactured in a manner similar to the method for preparing the pharmaceutical comprising the protein of the present invention described hereinabove.

Since the pharmaceutical preparation thus obtained is safe and low toxic, it can be administered to human or non-human mammal (e.g., rat, mouse, guinea pig, rabbit, sheep, swine, bovine, horse, cat, dog, monkey, etc.).

A dose of the compound or salts thereof may vary depending on target disease, subject to be administered, route for administration, etc.; for example, when the compound that inhibits the promoter activities to the DNA of the present invention is orally administered, the compound is administered to an adult patient (as 60 kg body weight) with breast cancer normally in a daily dose of about 0.1 to 100 mg, preferably about 1.0 to 50 mg and more preferably about 1.0 to 20 mg. In parenteral administration, a single dose of the compound varies depending on subject to be administered, target disease, etc. but when the compound of inhibiting the promoter activities to the DNA of the present invention is administered to an adult patient (as 60 kg) with breast cancer in the form of injectable preparation, it is advantageous to administer the compound intravenously in a daily dose of about 0.01 to about 30 mg, preferably about 0.1 to about 20 mg and more preferably about 0.1 to about 10 mg. For other animal species, the corresponding dose as converted per 60 kg weight can be administered.

As stated above, the non-human mammal deficient in expression of the DNA of the present invention is extremely useful for screening the compound or its salt that promotes or inhibits the promoter activities to the DNA of the present invention and, can greatly contribute to elucidation of causes for various diseases suspected of deficiency in expression of the DNA of the present invention and for the development of preventive/therapeutic agents for these diseases.

Also, a so-called transgenic animal (gene transferred animal) can be prepared by using a DNA containing the promoter region in the protein of the present invention, ligating genes encoding various proteins at the downstream and injecting the same into oocyte of an animal. It is thus possible to synthesize the protein therein specifically and study its activity in vivo. When an appropriate reporter gene is ligated to the promoter site described above and a cell line that expresses the gene is established, the resulting system can be utilized as the search system for a low molecular compound having the action of specifically promoting or inhibiting the in vivo productivity of the protein of the present invention itself.

In the specification and drawings, the codes of bases, amino acids, etc. are denoted in accordance with the IUPAC-IUB Commission on Biochemical Nomenclature or by the common codes in the art, examples of which are shown below. For amino acids that may have the optical isomer, L form is presented unless otherwise indicated.
- DNA: : deoxyribonucleic acid
- cDNA: : complementary deoxyribonucleic acid
- A: : adenine
- T: : thymine
- G: : guanine
- C: : cytosine
- RNA: :ribonucleic acid
- mRNA: : messenger ribonucleic acid
- dATP: : deoxyadenosine triphosphate
- dTTP: : deoxythymidine triphosphate
- dGTP: : deoxyguanosine triphosphate
- dCTP: : deoxycytidine triphosphate
- ATP: : adenosine triphosphate
- EDTA: : ethylenediaminetetraacetic acid
- SDS: : sodium dodecyl sulfate
- Gly: : glycine
- Ala: : alanine
- Val: : valine
- Leu: : leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagine
- Gln: : glutamine
- pGlu: : pyroglutamic acid
- See: : selenocysteine

Substituents, protecting groups and reagents generally used in this specification are presented as the codes below.
- Me: : methyl group
- Et: : ethyl group
- Bu: : butyl group
- Ph: : phenyl group
- TC: : thiazolidine-4(R)-carboxamido group
- Tos: : p-toluenesulfonyl
- CHO: : formyl
- Bzl: : benzyl
- Cl₂-Bzl: : 2,6-dichlorobenzyl
- Bom: : benzyloxymethyl
- Z: : benzyloxycarbonyl
- Cl-Z: : 2-chlorobenzyloxycarbonyl
- Br-Z: : 2-bromobenzyl oxycarbonyl
- Boc: : t-butoxycarbonyl
- DNP: : dinitrophenol
- Trt: : trityl
- Bum: : t-butoxymethyl
- Fmoc: : N-9-fluorenyl methoxycarbonyl
- HOBt: : 1-hydroxybenztriazole
- HOOBt: : 3,4-dihydro-3-hydroxy-4-oxo-1,2,3-benzotriazine
- HONB: : 1-hydroxy-5-norbornene-2,3-dicarboxyimide
- DCC: : N,N'-dicyclohexylcarbodiimide

The sequence identification numbers in the sequence listing of the specification indicates the following sequence, respectively.
[SEQ ID NO: 1] This shows the amino acid sequence of SUV39H1.
[SEQ ID NO: 2] This shows the base sequence of DNA encoding SUV39H1 having the amino acid sequence represented by SEQ ID NO: 1.
[SEQ ID NO: 3] This shows the base sequence of DNA containing the full-length gene encoding SUV39H1.
[SEQ ID NO: 4] This shows the base sequence of the antisense used in EXAMPLE 2.
[SEQ ID NO: 5] This shows the base sequence of the primer used in EXAMPLE 2.
[SEQ ID NO: 6] This shows the base sequence of the primer used in EXAMPLE 2.
[SEQ ID NO: 7] This shows the base sequence of the primer used in EXAMPLE 2.
[SEQ ID NO: 8] This shows the base sequence of the probe used in EXAMPLE 2.
[SEQ ID NO: 9] This shows the base sequence of the primer used in EXAMPLE 3.
[SEQ ID NO: 10] This shows the base sequence of the primer used in EXAMPLE 3.
[SEQ ID NO: 11] This shows the base sequence of the primer used in EXAMPLE 4.
[SEQ ID NO: 12] This shows the base sequence of the primer used in EXAMPLE 4.
[SEQ ID NO: 13] This shows the base sequence of the primer used in EXAMPLE 4.
[SEQ ID NO: 14] This shows the base sequence of the oligonucleotide for myc tag insertion used in EXAMPLE 4.
[SEQ ID NO: 15] This shows the base sequence of the oligonucleotide for myc tag insertion used in EXAMPLE 4.
[SEQ ID NO: 16] This shows the base sequence of the primer used in EXAMPLE 5.
[SEQ ID NO: 17] This shows the base sequence of the probe used in EXAMPLE 5.
[SEQ ID NO: 18] This shows the base sequence of the primer used in EXAMPLE 3.
[SEQ ID NO: 19] This shows the base sequence of the primer used in EXAMPLE 3.

### BEST MODE FOR CARRYING OUT THE INVENTION

The present invention is described in more detail below with reference to EXAMPLES, but is not deemed to limit the scope of the present invention thereto.

### EXAMPLE 1

In order to clarify the gene group in which its expression was specifically increased in the breast cancer tissue, the total RNAs (TABLE 1) extracted from one breast cancer tissue and other 23 normal tissues were used as materials and gene expression analysis was made using oligonucleotide microarray (Human Genome U95A, U95B, U95C, U95D, U95E; Affymetrix Inc.).

The experiment was carried out following the expression analysis technical manual of Affymetrix Inc. As a result, the expression of SUV39H1 gene was detected only in the breast cancer tissue. In all of the normal tissues analyzed, the values were less than the detection limit (TABLE 2).

**TABLE 1**

| RNA Samples under Gene Expression Analysis | |
|---|---|
| RNA-Extracted Tissue | Distribution Source |
| Breast cancer tissue (Patient No. 6) | BioClinical Partners |
| Fat | BioChain Institute |
| Skeletal muscle | Clontech |
| Heart | Clontech |
| Kidney | Clontech |
| Adrenal | Clontech |
| Liver | Clontech |
| Pancreas | Clontech |
| Spleen | Clontech |
| Trachea | Clontech |
| Lung | Clontech |
| Whole brain | Clontech |
| Cerebellum | Clontech |
| Thymus | Clontech |
| Mammary gland | Clontech |
| Salivary gland | Clontech |
| Stomach | Clontech |
| Rectum | BioChain Institute |
| Large intestine | BioChain Institute |
| Uterus | Clontech |
| Uterine cervix | BioChain Institute |

**TABLE 2**

| Comparison of SUV39H1 Gene Expression Level in Tissues | |
|---|---|
| Tissue | Gene Expression Level a |
| Breast cancer tissue (Patient No. 6) | 1.0 |
| Fat | ND |
| Skeletal muscle | ND |
| Heart | ND |
| Kidney | ND |
| Adrenal | ND |
| Liver | ND |
| Pancreas | ND |
| Spleen | ND |
| Trachea | ND |
| Lung | ND |
| Whole brain | ND |
| Cerebellum | ND |
| Thymus | ND |
| Mammary gland | ND |
| Salivary gland | ND |
| Stomach | ND |
| Rectum | ND |
| Large intestine | ND |
| Uterus | ND |
| Uterine cervix | ND |

| | |
|---|---|
| a: The gene expression level was standardized by taking as 1 the median value in the expression levels of all genes that the expression was detected with the oligonucleotide microarray. ND: not detected | |

### EXAMPLE 2

In order to analyze effects of the SUV39H1 gene with breast cancer-specifically observed expression on apoptosis, runs for transfection of SUV39H1 antisense oligonucleotide were performed.

First, the antisense (SEQ ID NO: 4) to the full-length sequence shown by SEQ ID NO: 3 was designed. Thereafter, phosphorothioated oligonucleotide was synthesized, purified on HPLC and used for the transfection test (Amersham Pharmacia Biotech). For control oligonucleotide, a reverse sequence (SEQ ID NO: 5) of the base sequence represented by SEQ ID NO: 4 was likewise phosphorothioated, purified on HPLC and used for the test (Amersham Pharmacia Biotech).

Breast cancer cell line MDA-MB-231 (In Vitro, 14 (11), 911-915, 1978, purchased from ATCC) was used as cells to be tested and on the day before transfecting the oligonucleotide, the cells were plated in 18 x 10³ on a 24-well plate (manufactured by Falcon). To transfect the oligonucleotide, OligofectAMINE (manufactured by Invitrogen) was used to follow its protocol. In accordance with the protocol of RNeasy mini kit (manufactured by Qiagen), RNA was extracted 7 hours after the transfection and cDNA was prepared using TaqMan Reverse Transcription Reagents (manufactured by Applied Biosystems). Using 2 primers (SEQ ID NO: 6 and SEQ ID NO: 7) and probe (SEQ ID NO: 8; Amersham Pharmacia Biotech), quantitative PCR was carried out by ABI PRISM TM 7700 Sequence Detector (manufactured by Applied Biosystems). On the other hand, the effects on apoptosis were examined by comparing with the control oligo-transfected sample on 3 days after the transfection of antisense oligonucleotide, using cell death detection ELISA (manufactured by Roche).

As a result, the expression (RNA) of SUV39H1 in 7 hours after the antisense transfection decreased to 70%, as compared to that when the control (reverse) oligonucleotide was transfected (100%), and apoptosis increased to 213% on 3 days after the transfection, as compared to control (100%).

The results revealed that SUV39H1 expressed not only cancer-specifically but was engaged in cancer cell growth and apoptosis.

### EXAMPLE 3

### (1) SUV39H1 expression vector

To perform the runs for expression of SUV39H1, the full-length gene was cloned.

Using 2 primers (SEQ ID NO: 18 and SEQ ID NO: 19) and Marathon Ready cDNA Library (manufactured by Clontech) as a template, PCR was carried out using Pfu polymerase (manufactured by Straragene). PCR was carried out by pre-treating 20 µl of a reaction mixture containing 2 µl of 10 x Pfu buffer, 0.4 µl of 10 mM each dNTP mixture (manufactured by Clontech), 0.8 µl each of the above-described 2 primers prepared in 20 mM, 0.5 µl of the template cDNA solution and 0.1 µl of Pfu polymerase at 94°C for 1 minute, then repeating the following cycle 42 times one set to include 94 °C for 1 minute, 57 °C for 1 minute and 72 °C for 2 minutes, and then reacting at 72 °C for 7 minutes. After PCR, the product was separated by agarose gel electrophoresis and the objective band was cut out. Using Gel Extraction kit (manufactured by Qiagen), the reaction product was purified. The purified product was then cloned to pCR-Blunt II-TOPO vector' (manufactured by Invitrogen) based on the protocol attached to Zero Blunt TOPO PCR Cloning Kit (Manufactured by Invitrogen) to acquire vector 1.

Next, using 2 primers (SEQ ID NO: 9 and SEQ ID NO: 10) and vector I obtained above as a template, PCR was carried out using Pfu polymerase (manufactured by Straragene). PCR was carried out by pre-treating 20 µl of a reaction mixture containing 2 µl of 10 x Pfu buffer, 0.4 µl of 10 mM each dNTP mixture (manufactured by Clontech), 0.8 µl each of the above-described 2 primers prepared in 20 mM, 0.5 µl of the template plasmid solution and 0.1 µl of Pfu polymerase at 94°C for 1 minute, then repeating the following cycle 25 times one set to include 94°C for 1 minute, 57 °C for 1 minute and 72°C for 2 minutes, and then reacting at 72 °C for 7 minutes.

After PCR, the product was separated by agarose gel electrophoresis and the objective band was cut out. Using Gel Extraction kit (manufactured by Qiagen), the reaction product was purified. Thereafter, addition A was made on the reaction product at the both ends thereof, according to the protocol attached to pcDNA3.1/V5 His TA Expression vector (manufactured by Invitrogen) to clone to the vector. The base sequence of the inserted fragment was confirmed to verify that there was no error in the sequence. Thus, SUV39H1 expression vector (vector 2) was acquired.

### (2) Effects on cancer suppressor gene p53

In order to examine the functions of SUV39H1, SUV39H1 was forcedly expressed in cancer cells to observe the effects.

Colorectal cancer-derived HCT116 (purchased from ATCC) was plated in 2 × 10⁴ on a 24-well plate (manufactured by Falcon). After incubation under conditions at 37 °C in 5% CO₂ for a day, the SUV39H1 expression vector (vector 2) (0.13 µg) acquired in (1) described above or pcDNA3.1 (manufactured by Invitrogen) (0.13 µg) for control was subjected to transfection.

To examine the action of SUV39H1 on p53, a reporter vector (manufactured by Clontech) (0.13 µg) inserted with p53 response element at the upstream of firefly luciferase gene was cotransfected together with the expression vector described in (1) above, and furthermore, pRL-TK (manufactured by Promega) (0.13 µg) was also cotransfected simultaneously to compensate for the transfection efficiency. As a transfection reagent, Fugene 6 (manufactured by Roche) was used and the protocol attached thereto was followed. Two days after the transfection, the firefly luciferase activity and the Renilla luciferase activity were assayed using Dual-Luciferase Reporter Assay System (manufactured by Promega). The firefly luciferase activity value measured was corrected by the Renilla luciferase activity value measured and comparison was made. When the value was made 100 upon the pcDNA3.1 transfection, it decreased to 65 when SUV39H1 was transfected. The results reveal that SUV39H1 suppressed the functions of cancer suppressor gene p53.

### EXAMPLE 4

### (1) Preparation of myc-tagged expression vector

In order to make the purification of SUV39H1 easy, the sequence was designed to have myc tag at the amino terminus. Using as a template vector I acquired in EXAMPLE 3 (1), PCR was carried out using Pfu polymerase (manufactured by Stratagene) in accordance with the protocol attached. Using 2 primers (SEQ ID NO: 11 and SEQ ID NO: 12) and the combination of 2 primers (SEQ ID NO: 11 and SEQ ID NO: 13) to prepare a variant deleted of 181 amino acid and thereafter, PCR was carried out as described above. PCR was carried out by pre-treating 20 µl of a reaction mixture containing 2 µl of 10 x Pfu buffer, 0.4 µl of 10 mM each dNTP mixture (manufactured by Clontech), 0.8 µl each of the above-described 2 primers prepared in 20 mM, 0.5 µl of the template plasmid solution and 0.1 µl of Pfu polymerase at 94°C for 1 minute, then repeating the following cycle 42 times one set to include 94°C for 1 minute, 57 °C for 1 minute and 72°C for 2 minutes, and then reacting at 72 °C for 7 minutes. After PCR, the product was separated by agarose gel electrophoresis and the objective band was cut out. Using Gel Extraction kit (manufactured by Qiagen), the reaction product was purified. The purified product was then digested with restriction enzymes EcoRI and XhoI, the product was again separated by agarose gel electrophoresis and the objective band was cut out. Nucleotide fragments having the base sequence represented by SEQ ID NO: 14 or SEQ ID NO: 15 including the myc tagged sequence were mixed, heated and then cooled. The reaction product was digested with restriction enzymes KpnI and EcoRI. After agarose gel electrophoresis, the enzymatic digestion product was purified using Gel Extraction kit (manufactured by Qiagen). The PCR product described above and the product obtained by digesting the myc tagged sequence with the restriction enzymes were ligated to pcDNA3.1(+) vector (manufactured by Invitrogen) digested with restriction enzymes KpnI and XhoI. The base sequence of the inserted fragment was confirmed to verify that there was no error in the sequence. Thus, the full-length SUV39H1 expression vector with Myc tag at the amino terminus and the SUV39H1 expression vector with Myc tag at the amino terminus, which was deleted of 181 amino acid and thereafter, could be acquired.

### (2) Expression of myc-tagged SUV39H1 protein

HeLa cells (purchased from ATCC) of 1 x 10⁵ were plated on a 35 mm dish (manufactured by Falcon). After incubation for a day, the myc-tagged SUV39H1 expression plasmid (1 µg) obtained in EXAMPLE 4 (1) described above was subjected to transfection in accordance with the protocol attached, using Fugene 6 (manufactured by Roche). On the day after the transfection, the culture medium was exchanged with a medium containing 500 µg/ml of G418 (manufactured by GIBCO). Thereafter the culture medium was exchanged with G418-containing medium every 3 other days to screen living cells. Next, the cells which became resistant to G418 were cultured on a 10 cm dish until confluent, and the nuclear extract was prepared using NE-PER (manufactured by Pierce). After the CERII solution was added, the mixture was ultrasonicated using a ultrasonic generator, otherwise following the protocol attached to NE-PER. The resulting nuclear extract protein solution (NER solution) was separated on SDS-PAGE, followed by western blotting. As a primary antibody; anti-myc antibody (manufactured by Invitrogene) was used and anti-mouse IgG-alkaline phosphatase conjugate (manufactured by Promega) was used as a secondary antibody. As a result, myc-tagged SUV39H1 protein of about 52 kDa could be identified.

### EXAMPLE 5

### (1) Preparation of His-tagged SUV39H1 expression vector

Using vector 2 obtained in EXAMPLE 3 (1) described above as a template together with 2 primers (SEQ ID NO: 16 and SEQ ID NO: 17), PCR was carried out using Pfu polymerase (manufactured by Stratagene) in accordance with the protocol attached. PCR was conducted by pre-treating 20 µl of a reaction mixture containing 2 µl of 10 x Pfu buffer, 1.6 µl of 2.5 mM each dNTP mixture (manufactured by Clontech), 0.2 µl each of the above-described 2 primers prepared in 50 mM, 0.5 µl of the template plasmid solution and 0.4 µl of Pfu polymerase at 94 °C for 1 minute, then repeating the following cycle 25 times one cycle set to include 94 °C for 1 minute, 57 °C for 1 minute and 72 °C for 2 minutes, and then reacting at 72 °C for 5 minutes. After the reaction, Advantage 2 polymerase (manufactured by Clontech) was added thereto. After reacting at 72 °C for 15 minutes, the product was electrophoresed on agarose gel and the objective band was cut out. Next, after the reaction product was purified using Gel Extraction kit (manufactured by Qiagen), ligation was performed according to the protocol attached to pQE30UA vector (manufactured by Qiagen) to transform to Escherichia coli JM109 (manufactured by Takara). It was confirmed that there was no error in the base sequence, and transformant JM109 bearing His-tagged SUV39H1 expression vector was acquired.

### (2) Expression of His-tagged SUV39H1 protein

The transformant obtained in (1) described above was incubated in 200 ml of CircleGrow medium, whereby the SUV39H1 protein was expressed. Using 1 mM of isopropylthiogalactopyranoside, expression induction was effected but the SUV39H1 protein was not obtained in the soluble fraction. Accordingly, the protein was solubilized under denaturation conditions and purified using Ni-NTA superflow (manufactured by Qiagen).

As a result, it was confirmed that the desired recombinant SUV39H1 protein was eluted in buffer E (A handbook for high-level expression and purification of 6 x His-tagged proteins, Qiagen). Next, for refolding of SUV39H1 in the denatured state, the eluate was dialyzed at 4°C through a dialysis membrane with fractionation molecular weight of 6000 to 8000 (manufactured by Spectrum Medical) sequentially to Buffer I for 2 hours, to Buffer II for 2.5 hours, to Buffer IIIf for 4 hours, to Buffer IV for 14 hours and to Buffer V for 2 hours. The precipitates generated during the course of dialysis were removed by centrifugation to acquire 0.04 mg of recombinant His-tagged SUV39H1 protein solution (dissolved in Buffer V).

### Buffer I:

0.2 M Tris-hydrochloride (pH 9.0), 3 mM 2-mercaptoethanol, 0.3 mM 2-hydroxyethyldisulfide, 3M urea and 0.1% Triton X-100

### Buffer II:

0.2 M Tris-hydrochloride (pH 9.0), 3 mM 2-mercaptoethanol, 0.3 mM 2-hydroxyethyldisulfide, 0.8 M urea and 0.1% Triton X-100

### Buffer III:

0.1 M Tris-hydrochloride (pH 8.5), 3 mM 2-mercaptoethanol, 0.3 mM 2-hydroxyethyldisulfide, 0.2 M urea; 0.1 M saccharose, 20 mM potassium chloride and 10 mM magnesium chloride

### Buffer IV:

50 mM Tris-hydrochloride (pH 8.5), 1 mM 2-mercaptoethanol, 0.1 mM 2-hydroxyethyldisulfide, 50 mM urea, 10 mM magnesium chloride, 20 mM potassium chloride and 250 mM saccharose

### Buffer V:

50 mM Tris-hydrochloride (pH 8.5), 20 mM potassium chloride, 10 mM magnesium chloride and 250 mM saccharose

### EXAMPLE 6

Using as an enzyme solution the protein solution obtained in EXAMPLE 4 (2) or EXAMPLE 5 (2), a test for screening the inhibitor is carried out.

On a streptoavidin-coated 96-well plate (manufactured by Perkin-Elmer), 50 µl of reaction buffer [50 mM Tris-hydrochloride (pH 8.5), 20 mM potassium chloride, 10 mM magnesium chloride, 250 mM saccharose and 10 mM 2-mercaptoethanol] containing 10 µl of the enzyme solution and 2 µl of DMF solution containing a test compound were mixed, and the mixture was allowed to stand at 37°C for 10 minutes. Next, 1 ng of biotin-labeled histone H3 peptide (manufactured by Upstate) and [³H] S-adenosylmethionine (manufactured by Amersham) (0.5 µCi) were added to and mixed with the mixture to initiate the reaction. After reacting at 37 °C for 3 hours, the reaction solution was discarded and then washed 3 times with wash buffer [phosphate buffer containing 0.05% Tween-20]. Then, 100 µl of liquid scintillator (manufactured by Wako Pure Chemical) was added and the radioactivity was counted with a liquid scintillation counter (manufactured by Wallac). When the activity obtained by adding the DMF solution containing no test compound is made 100, 50% inhibitory concentration (IC₅₀ value) can be determined. The compound which gives a lower. IC₅₀ value is screened as a compound that strongly inhibits the activities of the present invention.

### INDUSTRIAL APPLICABILITY

The protein used in the present invention is a diagnostic marker for cancer. The compound or its salt that regulates (preferably inhibits) the activities of the protein, the compound or its salt that regulates (preferably inhibits) the expression of a gene for the protein, the antibody of the present invention, the antisense nucleotide of the present invention, etc. can be used as safe pharmaceuticals, for example, preventive/therapeutic agents for cancer such as colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor, etc., apoptosis inducers, and the like.

## Claims

1. A preventive/therapeutic agent for cancer, comprising a compound or its salt that inhibits the activities of a protein containing the same or substantially the same amino acid sequence as the amino acid sequence represented by SEQ ID NO: 1, its partial peptide or a salt thereof.

2. A preventive/therapeutic agent for cancer, comprising a compound or its salt that inhibits the expression of a gene for a protein containing the same or substantially the same amino acid sequence as the amino acid sequence represented by SEQ ID NO: 1, its partial peptide or a salt thereof.

3. An antisense nucleotide containing a base sequence complementary or substantially complementary to the base sequence of a DNA encoding a protein having the same or substantially the same amino acid sequence as the amino acid sequence represented by SEQ ID NO: 1, its partial peptide or a salt thereof, or a part of the base sequence.

4. A preventive/therapeutic agent for cancer, comprising the antisense nucleotide according to claim 3.

5. A preventive/therapeutic agent for cancer, comprising an antibody to a protein containing the same or substantially the same amino acid sequence as the amino acid sequence represented by SEQ ID NO: 1, its partial peptide or a salt thereof.

6. The preventive/therapeutic agent for cancer according to claims 1 through 5, wherein the cancer is colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor.

7. A diagnostic product comprising an antibody to a protein containing the same or substantially the same amino acid sequence as the amino acid sequence represented by SEQ ID NO: 1, its partial peptide or a salt thereof.

8. A diagnostic product comprising a DNA encoding a protein containing the same or substantially the same amino acid sequence as the amino acid sequence represented by SEQ ID NO: 1, its partial peptide or a salt thereof.

9. The diagnostic product according to claim 7 or 8, wherein the cancer is colorectal cancer, breast cancer, lung cancer, prostate cancer, esophageal cancer, gastric cancer, liver cancer, biliary tract cancer, spleen cancer, renal cancer, bladder cancer, uterus cancer, ovarian cancer, testicular cancer, thyroid cancer, pancreatic cancer, brain tumor or blood tumor.

10. A preventive/therapeutic agent for cancer, comprising a compound having a regulatory action on the histone methyltransferase activity, or a salt thereof.

11. A preventive/therapeutic agent for cancer, comprising a compound having a histone methyltransferase expression regulatory action, or a salt thereof.

12. A method of screening a preventive/therapeutic agent for cancer, which comprises using a protein containing the same or substantially the same amino acid sequence as the amino acid sequence represented by SEQ ID NO: 1, its partial peptide or a salt thereof.

13. A kit for screening a preventive/therapeutic agent for cancer, comprising a protein containing the same or substantially the same amino acid sequence as the amino acid sequence represented by SEQ ID NO: 1, its partial peptide or a salt thereof.

14. A method of screening a preventive/therapeutic agent for cancer, which comprises using a DNA encoding a protein containing the same or substantially the same amino acid sequence as the amino acid sequence represented by SEQ ID NO: 1, its partial peptide or a salt thereof.

15. A kit for screening a preventive/therapeutic agent for cancer, comprising a DNA encoding a protein containing the same or substantially the same amino acid sequence as the amino acid sequence represented by SEQ ID NO: 1, its partial peptide or a salt thereof.

16. A preventive/therapeutic agent for cancer, which is obtainable by using the screening method according to claim 12 or 14 or the screening kit according to claim 13 or 15.

17. An apoptosis inducer comprising a compound or its salt that inhibits the activities of a protein containing the same or substantially the same amino acid sequence as the amino acid sequence represented by SEQ ID NO: 1, its partial peptide or a salt thereof.

18. An apoptosis inducer comprising a compound or its salt that inhibits the expression of a gene for a protein containing the same or substantially the same amino acid sequence as the amino acid sequence represented by SEQ ID NO: 1, its partial peptide or a salt thereof.

19. A method of screening an apoptosis inducer, which comprises using a protein containing the same or substantially the same amino acid sequence as the amino acid sequence represented by SEQ ID NO: 1, its partial peptide or a salt thereof.

20. A method of screening an apoptosis inducer, which comprises using a DNA encoding a protein containing the same or substantially the same amino acid sequence as the amino acid sequence represented by SEQ ID NO: 1, its partial peptide or a salt thereof.

21. A method of preventing/treating cancer, which comprises administering to a mammal an effective amount of a compound or its salt that inhibits the activities of a protein containing the same or substantially the same amino acid sequence as the amino acid sequence represented by SEQ ID NO: 1, its partial peptide or a salt thereof, or a compound or its salt that inhibits the expression of a gene for the protein, its partial peptide or a salt thereof.

22. Use of a compound or its salt that inhibits the activities of a protein containing the same or substantially the same amino acid sequence as the amino acid sequence represented by SEQ ID NO: 1, its partial peptide or a salt thereof, or a compound or its salt that inhibits the expression of a gene for the protein, its partial peptide or a salt thereof.


SEQUENCE LISTING

<110> Takeda Chemical Industries, Ltd.

<120> Preventives/Remedies For Cancer

<130> P02-0148PCT

<150> JP2001-398220
<151> 2001-12-27

<160> 19

<210> 1
<211> 412
<212> PRT
<213> Homo Sapiens

<400> 1
Met Ala Glu Asn Leu Lys Gly Cys Ser Val Cys Cys Lys Ser Ser Trp
                5                   10                  15
Asn Gln Leu Gln Asp Leu Cys Arg Leu Ala Lys Leu Ser Cys Pro Ala
            20                  25                  30
Leu Gly Ile Ser Lys Arg Asn Leu Tyr Asp Phe Glu Val Glu Tyr Leu
        35                  40                  45
Cys Asp Tyr Lys Lys Ile Arg Glu Gln Glu Tyr Tyr Leu Val Lys Trp
    50                  55                  60
Arg Gly Tyr Pro Asp Ser Glu Ser Thr Trp Glu Pro Arg Gln Asn Leu
65                  70                  75                  80
Lys Cys Val Arg Ile Leu Lys Gln Phe His Lys Asp Leu Glu Arg Glu
                85                  90                  95
Leu Leu Arg Arg His His Arg Ser Lys Thr Pro Arg His Leu Asp Pro
            100                 105                 110
Ser Leu Ala Asn Tyr Leu Val Gln Lys Ala Lys Gln Arg Arg Ala Leu
        115                 120                 125
Arg Arg Trp Glu Gln Glu Leu Asn Ala Lys Arg Ser His Leu Gly Arg
    130                 135                 140
Ile Thr Val Glu Asn Glu Val Asp Leu Asp Gly Pro Pro Arg Ala Phe
145                 150                 155                 160
Val Tyr Ile Asn Glu Tyr Arg Val Gly Glu Gly Ile Thr Leu Asn Gln
                165                 170                 175
Val Ala Val Gly Cys Glu Cys Gln Asp Cys Leu Trp Ala Pro Thr Gly
            180                 185                 190
Gly Cys Cys Pro Gly Ala Ser Leu His Lys Phe Ala Tyr Asn Asp Gln
        195                 200                 205
Gly Gln Val Arg Leu Arg Ala Gly Leu Pro Ile Tyr Glu Cys Asn Ser
    210                 215                 220
Arg Cys Arg Cys Gly Tyr Asp Cys Pro Asn Arg Val Val Gln Lys Gly
225                 230                 235                 240
Ile Arg Tyr Asp Leu Cys Ile Phe Arg Thr Asp Asp Gly Arg Gly Trp
                245                 250                 255
Gly Val Arg Thr Leu Glu Lys Ile Arg Lys Asn Ser Phe Val Met Glu
            260                 265                 270
Tyr Val Gly Glu Ile Ile Thr Ser Glu Glu Ala Glu Arg Arg Gly Gln
        275                 280                 285
Ile Tyr Asp Arg Gln Gly Ala Thr Tyr Leu Phe Asp Leu Asp Tyr Val
    290                 295                 300
Glu Asp Val Tyr Thr Val Asp Ala Ala Tyr Tyr Gly Asn Ile Ser His
305                 310                 315                 320
Phe Val Asn His Ser Cys Asp Pro Asn Leu Gln Val Tyr Asn Val Phe
                325                 330                 335
Ile Asp Asn Leu Asp Glu Arg Leu Pro Arg Ile Ala Phe Phe Ala Thr
            340                 345                 350
Arg Thr Ile Arg Ala Gly Glu Glu Leu Thr Phe Asp Tyr Asn Met Gln
        355                 360                 365
Val Asp Pro Val Asp Met Glu Ser Thr Arg Met Asp Ser Asn Phe Gly

370                 375                 380
Leu Ala Gly Leu Pro Gly Ser Pro Lys Lys Arg Val Arg Ile Glu Cys
385                 390                 395                 400
Lys Cys Gly Thr Glu Ser Cys Arg Lys Tyr Leu Phe
                405                 410

<210> 2
<211> 1236
<212> DNA
<213> Homo Sapiens

<400> 2
atggcggaaa atttaaaagg ctgcagcgtg tgttgcaagt cttcttggaa tcagctgcag      60
gacctgtgcc gcctggccaa gctctcctgc cctgccctcg gtatctctaa gaggaacctc     120
tatgactttg aagtcgagta cctgtgcgat tacaagaaga tccgcgaaca ggaatattac     180
ctggtgaaat ggcgtggata tccagactca gagagcacct gggagccacg gcagaatctc     240
aagtgtgtgc gtatcctcaa gcagttccac aaggacttag aaagggagct gctccggcgg     300
caccaccggt caaagacccc ccggcacctg gacccaagct tggccaacta cctggtgcag     360
aaggccaagc agaggcgggc gctccgtcgc tgggagcagg agctcaatgc caagcgcagc     420
catctgggac gcatcactgt agagaatgag gtggacctgg acggccctcc gcgggccttc     480
gtgtacatca atgagtaccg tgttggtgag ggcatcaccc tcaaccaggt ggctgtgggc     540
tgcgagtgcc aggactgtct gtgggcaccc actggaggct gctgcccggg ggcgtcactg     600
cacaagtttg cctacaatga ccagggccag gtgcggcttc gagccgggct gcccatctac     660
gagtgcaact cccgctgccg ctgcggctat gactgcccaa atcgtgtggt acagaagggt     720
atccgatatg acctctgcat cttccggacg gatgatgggc gtggctgggg cgtccgcacc     780
ctggagaaga ttcgcaagaa cagcttcgtc atggagtacg tgggagagat cattacctca     840
gaggaggcag agcggcgggg ccagatctac gaccgtcagg gcgccaccta cctctttgac     900
ctggactacg tggaggacgt gtacaccgtg gatgccgcct actatggcaa catctcccac     960
tttgtcaacc acagttgtga ccccaacctg caggtgtaca acgtcttcat agacaacctt    1020
gacgagcggc tgccccgcat cgctttcttt gccacaagaa ccatccgggc aggcgaggag    1080
ctcacctttg attacaacat gcaagtggac cccgtggaca tggagagcac ccgcatggac    1140
tccaactttg gcctggctgg gctccctggc tcccctaaga agcgggtccg tattgaatgc    1200
aagtgtggga ctgagtcctg ccgcaaatac ctcttc                              1236

<210> 3
<211> 2754
<212> DNA
<213> Homo Sapiens

<400> 3
ggcacgaggc gcgaggccgg ctaggcccga atgtcgttag ccgtggggaa agatggcgga      60
aaatttaaaa ggctgcagcg tgtgttgcaa gtcttcttgg aatcagctgc aggacctgtg     120
ccgcctggcc aagctctcct gccctgccct cggtatctct aagaggaacc tctatgactt     180
tgaagtcgag tacctgtgcg attacaagaa gatccgcgaa caggaatatt acctggtgaa     240
atggcgtgga tatccagact cagagagcac ctgggagcca cggcagaatc tcaagtgtgt     300
gcgtatcctc aagcagttcc acaaggactt agaaagggag ctgctccggc ggcaccaccg     360
gtcaaagacc ccccggcacc tggacccaag cttggccaac tacctggtgc agaaggccaa     420
gcagaggcgg gcgctccgtc gctgggagca ggagctcaat gccaagcgca gccatctggg     480
acgcatcact gtagagaatg aggtggacct ggacggccct ccgcgggcct tcgtgtacat     540
caatgagtac cgtgttggtg agggcatcac cctcaaccag gtggctgtgg gctgcgagtg     600
ccaggactgt ctgtgggcac ccactggagg ctgctgcccg ggggcgtcac tgcacaagtt     660
tgcctacaat gaccagggcc aggtgcggct tcgagccggg ctgcccatct acgagtgcaa     720
ctcccgctgc cgctgcggct atgactgccc aaatcgtgtg gtacagaagg gtatccgata     780
tgacctctgc atcttccgca cggatgatgg gcgtggctgg ggcgtccgca ccctggagaa     840
gattcgcaag aacagcttcg tcatggagta cgtgggagag atcattacct cagaggaggc     900
agagcggcgg ggccagatct acgaccgtca gggcgccacc tacctctttg acctggacta     960
cgtggaggac gtgtacaccg tggatgccgc ctactatggc aacatctccc actttgtcaa    1020
ccacagttgt gaccccaacc tgcaggtgta caacgtcttc atagacaacc ttgacgagcg    1080
gctgccccgc atcgctttct ttgccacaag aaccatccgg gcaggcgagg agctcacctt    1140
tgattacaac atgcaagtgg accccgtgga catggagagc acccgcatgg actccaactt    1200
tggcctggct gggctccctg gctcccctaa gaagcgggtc cgtattgaat gcaagtgtgg    1260
gactgagtcc tgccgcaaat acctcttcta gcccttagaa gtctgaggcc agactgactg    1320
agggggcctg aagctacatg cacctccccc actgctgccc tcctgtcgag aatgactgcc    1380
agggcctcgc ctgcctccac ctgcccccac ctgctcctac ctgctctacg ttcagggctg    1440
tggccgtggt gaggaccgac tccaggagtc ccctttccct gtcccagccc catctgtggg    1500
ttgcacttac aaacccccac ccaccttcag aaatagtttt tcaacatcaa gactctctgt    1560
cgttgggatt catggcctat taaggaggtc caaggggtga gtcccaaccc agccccagaa    1620
tatatttgtt tttgcacctg cttctgcctg gagattgagg ggtctgctgc aggcctcctc    1680

cctgctgccc caaaggtatg gggaagcaac cccagagcag gcagacatca gaggccagag 1740
tgcctagccc gacatgaagc tggttcccca accacagaaa ctttgtacta gtgaaagaaa 1800
gggggtccct gggctacggg ctgaggctgg tttctgctcg tgcttacagt gctgggtagt 1860
gttggcccta agagctgtag ggtctcttct tcagggctgc atatctgaga agtggatgcc 1920
cacatgccac tggaagggaa gtgggtgtcc atgggccact gagcagtgag aggaaggcag 1980
tgcagagctg gccagccctg gaggtaggct gggaccaagc tctgccttca cagtgcagtg 2040
aaggtaccta gggctcttgg gagctctgcg gttgctaggg gccctgacct ggggtgtcat 2100
gaccgctgac accactcaga gctggaacca agatctagat agtccgtaga tagcacttag 2160
gacaagaatg tgcattgatg gggtggtgat gaggtgccag gcactgggta gagcacctgg 2220
tccacgtgga ttgtctcagg gaagccttga aaaccacgga ggtggatgcc aggaaagggc 2280
ccatgtggca gaaggcaaag tacaggccaa gaattggggg tgggggagat ggcttcccca 2340
ctatgggatg acgaggcgag agggaagccc ttgctgcctg ccattcccag accccagccc 2400
tttgtgctca ccctggttcc actggtctca aaagtcacct gcctacaaat gtacaaaagg 2460
cgaaggttct gatggctgcc ttgctccttg ctcccccacc ccctgtgagg acttctctag 2520
gaagtccttc ctgactacct gtgcccagag tgcccctaca tgagactgta tgccctgcta 2580
tcagatgcca gatctatgtg tctgtctgtg tgtccatccc gccgaccccc cagactaacc 2640
tccaggcatg gactgaatct ggttctcctc ttgtacaccc ctcaacccta tgcagcctgg 2700
agtgggcatc aataaaatga actgtcgact gaaaaaaaaa aaaaaaaaaa aaaa 2754

<210> 4
<211> 20
<212> DNA
<213> Artificial Sequence

<220>
<223> Primer

<400> 4
atgcgtccca gatggctgcg 20

<210> 5
<211> 20
<212> DNA
<213> Artificial Sequence

<220>
<223> Primer

<400> 5
gcgtcggtag accctgcgta 20

<210> 6
<211> 18
<212> DNA
<213> Artificial Sequence

<220>
<223> Primer

<400> 6
aaccttgacg agcggctg 18

<210> 7
<211> 14
<212> DNA
<213> Artificial Sequence

<220>
<223> Primer

<400> 7
tcgcctgccg gatg 14

<210> 8
<211> 23
<212> DNA
<213> Artificial Sequence

<220>
<223> Probe

<400> 8
ccgcatcgct ttctttgcca caa                                              23

<210> 9
<211> 24
<212> DNA
<213> Artificial Sequence

<220>
<223> Primer

<400> 9
ggaaagatgg cggaaaattt aaaa                                             24

<210> 10
<211> 24
<212> DNA
<213> Artificial Sequence

<220>
<223> Primer

<400> 10
gggctagaag aggtatttgc ggca                                             24

<210> 11
<211> 31
<212> DNA
<213> Artificial Sequence

<220>
<223> Primer

<400> 11
ccggaattcg cggaaaattt aaaaggctgc a                                     31

<210> 12
<211> 29
<212> DNA
<213> Artificial Sequence

<220>
<223> Primer

<400> 12
ccgctcgagc tagaagaggt atttgcggc                                        29

<210> 13
<211> 32
<212> DNA
<213> Artificial Sequence

<220>
<223> Primer

<400> 13
ccgctcgagc tagcccacag ccacctggtt ga                                    32

<210> 14
<211> 113
<212> DNA
<213> Artificial Sequence

<220>
<223> Primer

<400> 14
ggggtaccaa gatggagcag aaactcatct ctgaagagga tctggagcag aaactcatct    60
ctgaagagga tctggagcag aaactcatct ctgaagagga tctggaattc cgg          113

<210> 15
<211> 113
<212> DNA
<213> Artificial Sequence

<220>
<223> Primer

<400> 15
ccggaattcc agatcctctt cagagatgag tttctgctcc agatcctctt cagagatgag    60
tttctgctcc agatcctctt cagagatgag tttctgctcc atcttggtac ccc          113

<210> 16
<211> 24
<212> DNA
<213> Artificial Sequence

<220>
<223> Primer

<400> 16
tgtgtgcgta tcctcaagca gttc                                          24

<210> 17
<211> 24
<212> DNA
<213> Artificial Sequence

<220>
<223> Primer

<400> 17
ctagaagagg tatttgcggc agga                                          24

<210> 18
<211> 24
<212> DNA
<213> Artificial Sequence

<220>
<223> Primer

<400> 18
gtcgttagcc gtggggaaag atgg                                          24

<210> 19
<211> 24
<212> DNA
<213> Artificial Sequence

<220>
<223> Primer

<400> 19
atatgcagcc ctgaagaaga gacc                                          24